# EUROPÄISCHE PATENTSCHRIFT

(11) **EP 0 835 324 B1**
(45) Veröffentlichungstag und Bekanntmachung des Hinweises auf die Patenterteilung: **24.04.2002**
(21) Anmeldenummer: 96921885.8
(22) Anmeldetag: 27.06.1996
(51) Int. Cl.: C12Q 1/68

(54) **MIKROSATELLITENMARKER FÜR PFLANZEN DER SPEZIES TRITICUM AESTIVUM SOWIE DES TRIBUS TRITICEAE UND IHRE VERWENDUNG**
MICROSATELLITE MARKERS FOR PLANTS OF THE SPECIES TRITICUM AESTIVUM AND TRIBE TRITICEAE AND THE USE OF SAID MARKERS
MARQUEURS DE MICROSATELLITES POUR VEGETAUX DE L'ESPECE TRITICUM AESTIVUM ET DE LA TRIBU TRITICEAE ET LEUR UTILISATION

(30) Priorität: 28.06.1995 DE 19525284
(43) Veröffentlichungstag der Anmeldung: 15.04.1998
(73) Patentinhaber: INSTITUT FÜR PFLANZENGENETIK UND KULTURPFLANZENFORSCHUNG, 06466 Gatersleben (DE)
(72) Erfinder: RÖDER, Marion, D-06507 Rieder (DE); PLASCHKE, Jens, D-01662 Meissen (DE); GANAL, Martin, D-06507 Rieder (DE)
(74) Vertreter: Baumbach, Friedrich
(86) Internationale Anmeldenummer: DE9601185
(87) Internationale Veröffentlichungsnummer: WO9701567

(56) Entgegenhaltungen:
- MOLECULAR AND GENERAL GENETICS, Bd. 246, Nr. 3, 6.Februar 1995, Seiten 327-33, XP000605158 RÖDER M ET AL: "Abundance, variability and chromosal location of microsatellites in wheat" in der Anmeldung erwähnt
- THEORETICAL AND APPLIED GENETICS, Bd. 90, Nr. 2, 1.Februar 1995, Seiten 247-252, XP000604132 DEVOS K ET AL: "Application of two microsatellite sequences in wheat storage proteins as molecular markers"
- HORTSCIENCE, Bd. 30, Nr. 1, 1.Januar 1995, Seiten 109-112, XP000604253 SHARON D ET AL: "DNA fingerprints in plants using simple-sequence repeat and minisatellite probes"
- GENOME, Bd. 36, Oktober 1993, Seiten 884-889, XP000569548 SENIOR M ET AL: "Mapping maize microsatellites and polymerase chain reaction confirmation of the targeted repeats using a ct primer"
- THE PLANT JOURNAL, Bd. 3, Nr. 1, 1.Januar 1993, Seiten 175-82, XP000608178 MORGANTE M ET AL: "PCR-amplified microsatellites as markers in plant genetics"
- EUPHYTICA, Bd. 89, Nr. 1, 1.Januar 1996, Seiten 33-40, XP000604254 PLASCHKE ET AL: "The use of wheat anaploids for chromosomal assignment of microsatellite loci"
- BRYAN G.J. ET AL.: 'Isolation and characterization of microsatellites from hexaploid bread wheat' THEOR APPL GENET Bd. 94, 1997, Seiten 557 - 563

## Beschreibung

Die Erfindung betrifft neuartige genetische Marker für Weizen (*Triticum aestivum* L.) und nahe verwandte Spezies (Tribus Triticeae) sowie ihre Verwendung.

Die am weitesten verbreiteten bekannten genetischen Marker auf DNS-Basis sind die sogenannten Restriktionsfragmentlängenpolymorphismen (RFLP)-Marker. Zur Anwendung dieser Marker wird genomische DNS mit Restriktionsenzymen verdaut, auf Agarosegelen aufgetrennt und auf Nylonmembranen transferiert (Southernblot). Durch Hybridisierung mit radioaktiv markierten DNS-Sonden werden spezifische Fragmente detektiert. Beim Auftreten von Mutationen im Bereich der verwendeten Restriktionsenzyme oder kleineren Deletionen/Insertionen werden Polymorphismen zwischen verschiedenen Linien gefunden, welche stabil und meist kodominant vererbt werden. Die Verwendung von RFLP-Markern in hexaploiden Kulturweizen ist nur beschränkt möglich, da auf diese Weise in Weizen nur sehr wenig Polymorphismus detektiert wird.

Es wurde bereits beschrieben, daß Mikrosatellitenmarker zwischen verschiedenen Weizenlinien signifikant mehr Polymorphismus detektieren als RFLP-Marker. Dies ist vor allem auf das Auftreten multipler Allele je Locus zurückzuführen (Röder et al., Mol Gen Genet (1995) 246, 327-333). Daneben ist bekannt, daß Mikrosatellitenmarker den Vorteil haben, daß sie über PCR detektiert werden und daher leichter große Mengen an Proben analysiert werden können. Devos et al. beschreiben in Theor. Appl. Genet. 90: 247-252 Methoden zur Isolation von zwei Mikrosatellitenmarkern, die jedoch nicht geeignet sind, eine große Anzahl an Mikrosatellitenmarkern zu isolieren, da die vorhandenen Mikrosatelliten enthaltenden Weizensequenzen in Sequenzdatenbanken bei weitem nicht ausreichend waren, um eine große Anzahl von Markern zu entwickeln.

Die Aufgabe der Erfindung besteht darin, weitere Mikrosatellitenmarker zur genetischen Analyse von Pflanzen der Spezies Triticum aestivum bereitzustellen, die sich durch einen höheren Grad an DNS-Polymorphismus auszeichnen als andere bisher für das Weizengenom entwickelte molekulare Sonden.

Die Aufgabe wird gemäß der Ansprüche 1 bis 6 realisiert. Die erfindungsgemäßen Marker basieren auf der Amplifikation bestimmter hypervariabler Genomabschnitte, den sogenannten Mikrosatelliten, mit Hilfe der Polymerasekettenreaktion (PCR). Zur spezifischen Amplifikation werden für jeden Mikrosatelliten-Locus zwei Primer gemäß Anspruch 1, jeweils links und rechts in den flankierenden Sequenzen benötigt. Diese Primer sind im Durchschnitt 20 ± 3 Basen lang und durch ihre Sequenzen definiert. Ein Mikrosatellitenmarker ist im Prinzip eine sequence tagged site (STS), welche durch zwei spezifische Primer definiert ist. Diese Primer flankieren, jeweils links und rechts, eine sogenannte Mikrosatellitensequenz. Eine Mikrosatellitensequenz ist definiert als tandemrepetitive Wiederholung einer Di-, Tri- oder Tetranukleotidsequenz, beispielsweise (GA)ₙ, wobei n≥10. Es treten auch zusammengesetzte Mikrosatellitensequenzen auf, beispielsweise (GT)ₙ(AT)ₙ, sowie imperfekte Sequenzen, bei welchen einzelne Basen mutiert sind, beispielsweise (GA)ₙCA(GA)ₙ. Zwischen verschiedenen Linien und Sorten kommt es zu Variationen der Anzahl der Repeats an einem bestimmten Locus. Dies führt nach Amplifikation des Mikrosatelliten mittels der spezifischen Primer in den flankierenden Sequenzen zu PCR-Produkten verschiedener Länge und damit zu Polymorphismen. Diese Polymorphismen werden stabil vererbt und können daher als genetische Marker verwendet werden. In manchen Fällen treten auch Nullallele (kein sichtbares Fragment) auf, wenn Mutationen innerhalb der Bindungsstelle für die Primer vorhanden sind.

Die Auftrennung und Detektion der erhaltenen PCR-Produkte kann mit verschiedenen technischen Varianten durchgeführt werden. Für die Auftrennung der Fragmente können hochauflösende Agarosegele, native Polyacrylamidgele oder denaturierende Polyacrylamidgele (= Sequenziergele) verwendet werden. Die Detektion der Fragmente kann je nach Trennungssystem über Ethidiumbromidfärbung, Silberfärbung oder bei radioaktiver Markierung der PCR-Fragmente über Autoradiographie erfolgen. Eine weitere sehr effektive Variante der Auftrennung und Detektion besteht im Einsatz eines automatischen Sequenziergerätes mit farbstoffbzw.fluoreszenzmarkierten Primern. Hierzu ist erforderlich, einen Primer aus jedem Mikrosatelliten-Primerpaar farbstoff- bzw. fluoreszenzmarkiert zu synthetisieren. Aus der PCR-Amplifikation resultiert ein markiertes Produkt, welches von dem Sequenziergerät detektiert werden kann. Dabei werden für jede Probe farbstoff- bzw. fluoreszenzmarkierte Größenstandards in derselben Spur mit aufgetrennt. Eine spezielle Software erlaubt es danach, die absolute Größe jedes aufgetrennten Fragmentes zu berechnen und somit auch Fragmente zwischen verschiedenen Gelläufen zu vergleichen. Mit dieser Methode können pro Tag mehrere hundert Proben weitgehend automatisch analysiert werden.

Erfindungsgemäß werden Mikrosatellitenmarker bereitgestellt, die folgende Primerpaare mit zugeordneten Mikrosatellitensequenzen bzw. eine Anzahl davon enthalten und die Loci von allen Chromosomen des Weizengenoms amplifizieren und daher zur Genmarkierung Verwendung finden.

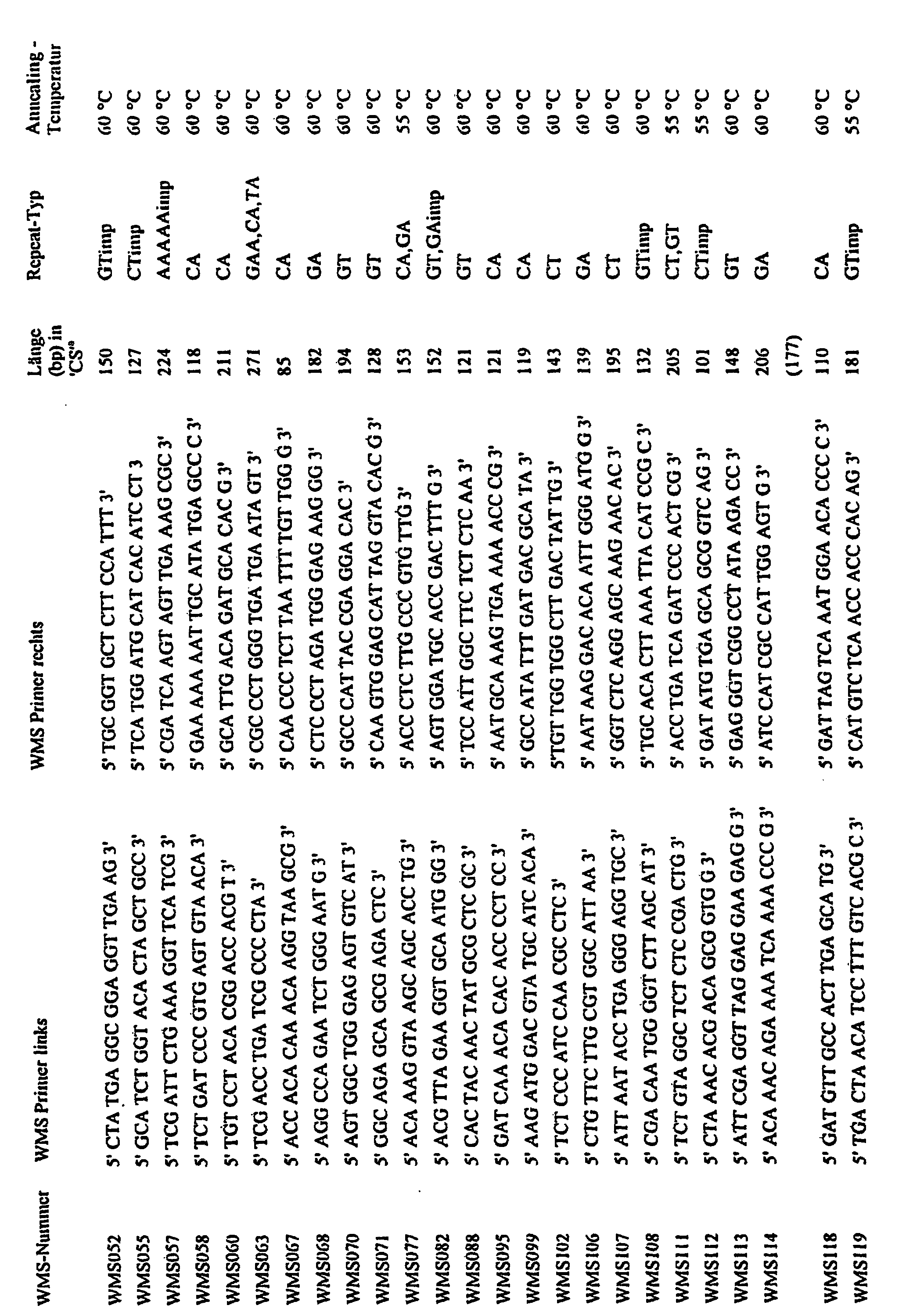

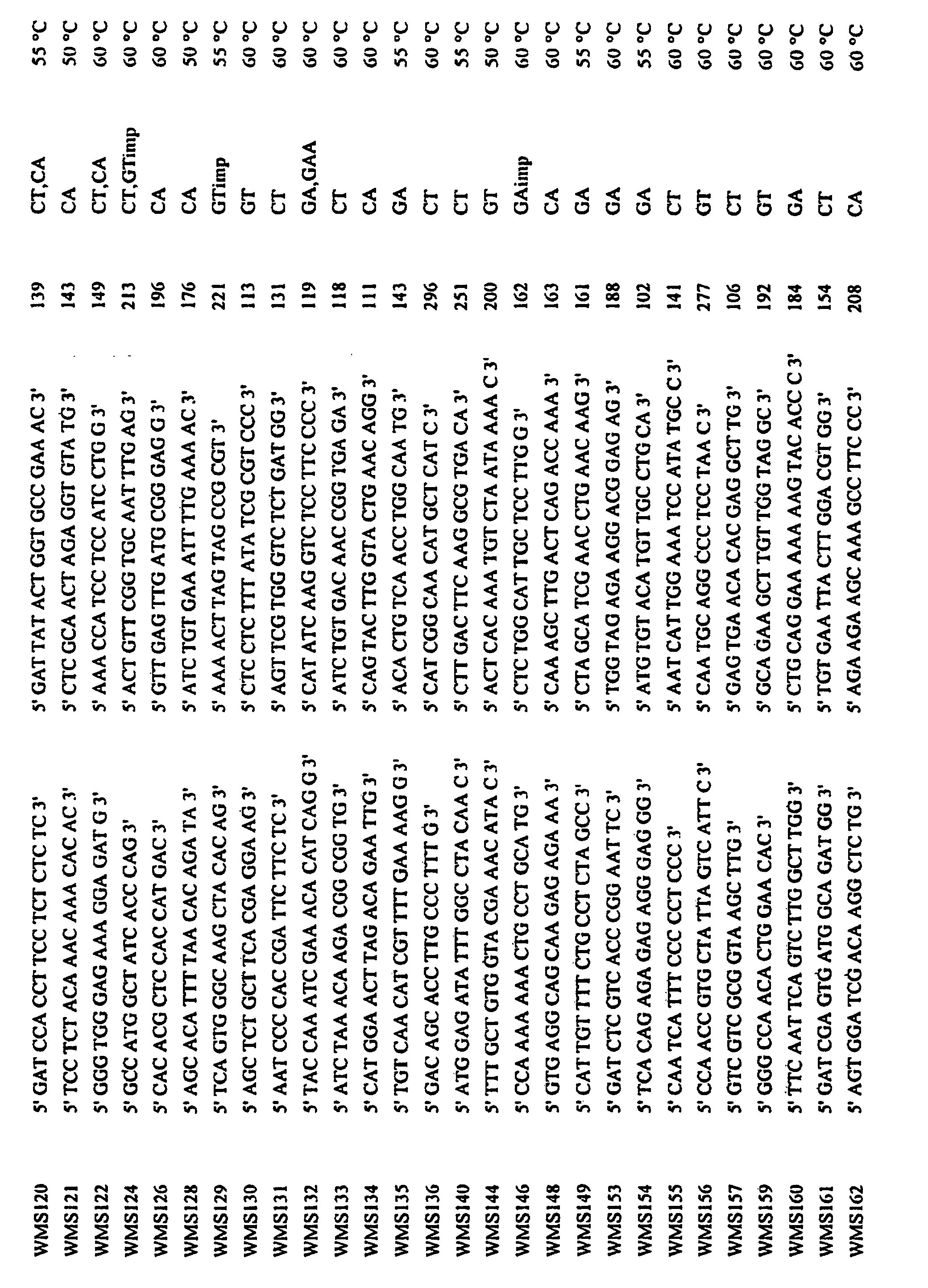

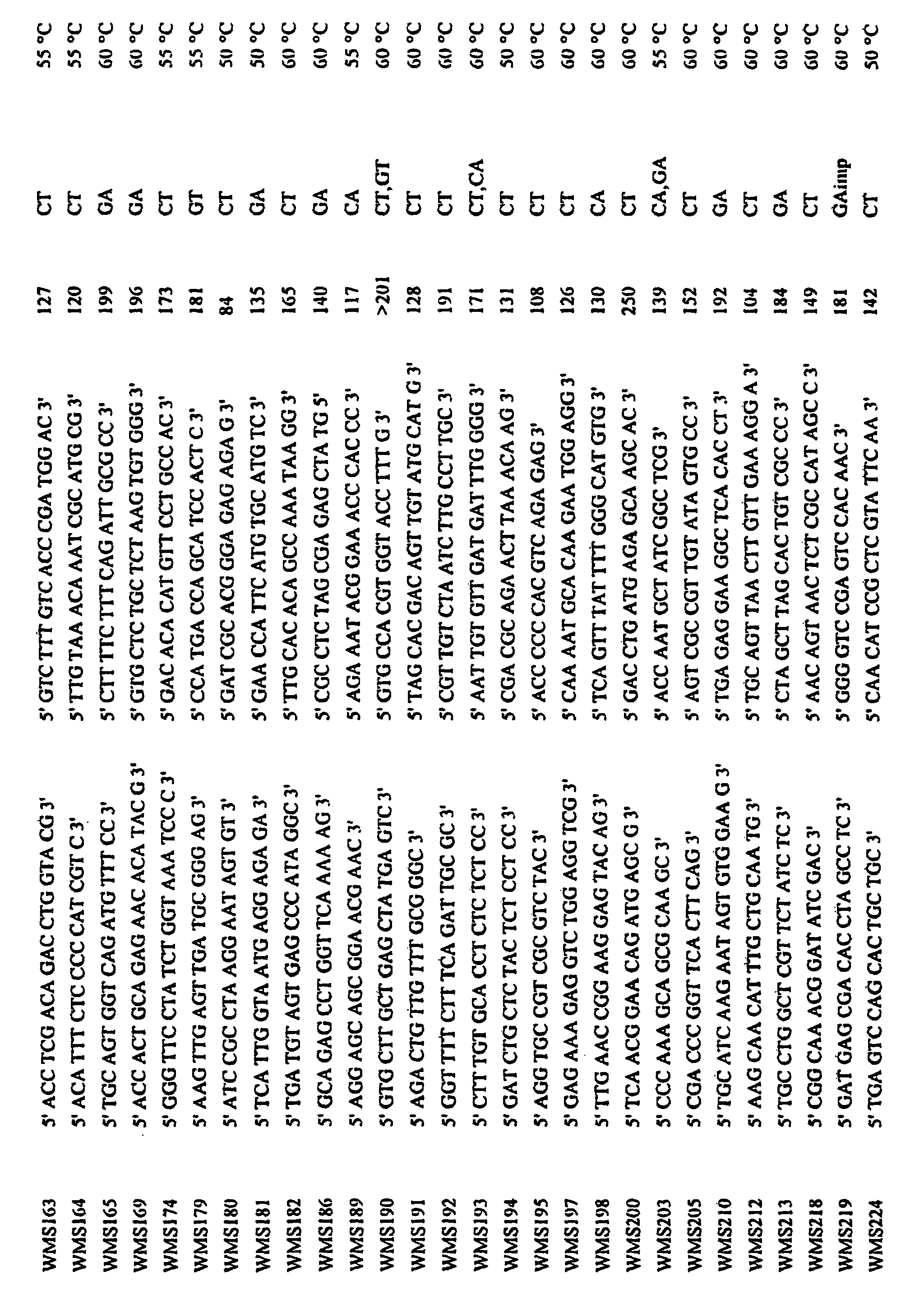

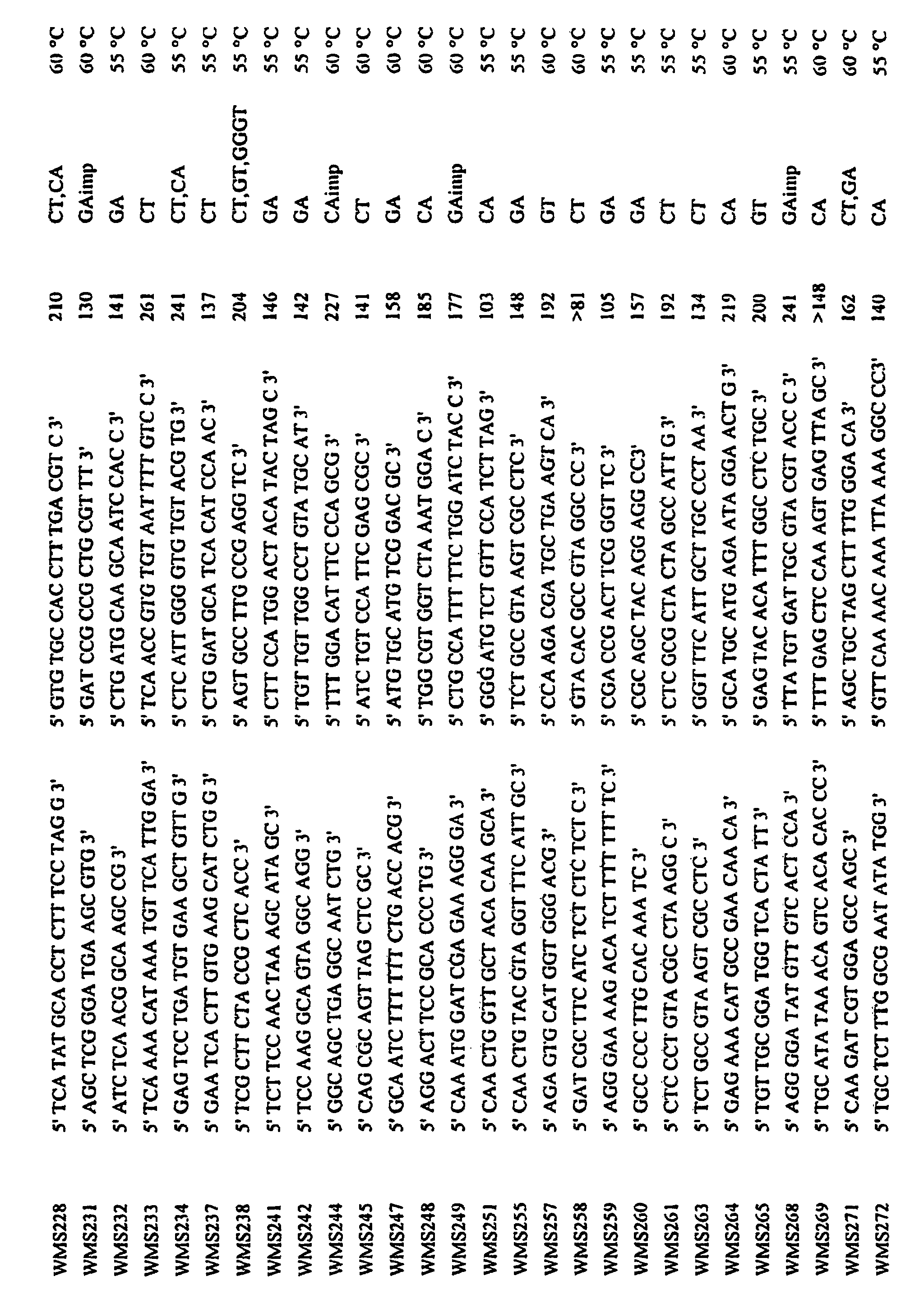

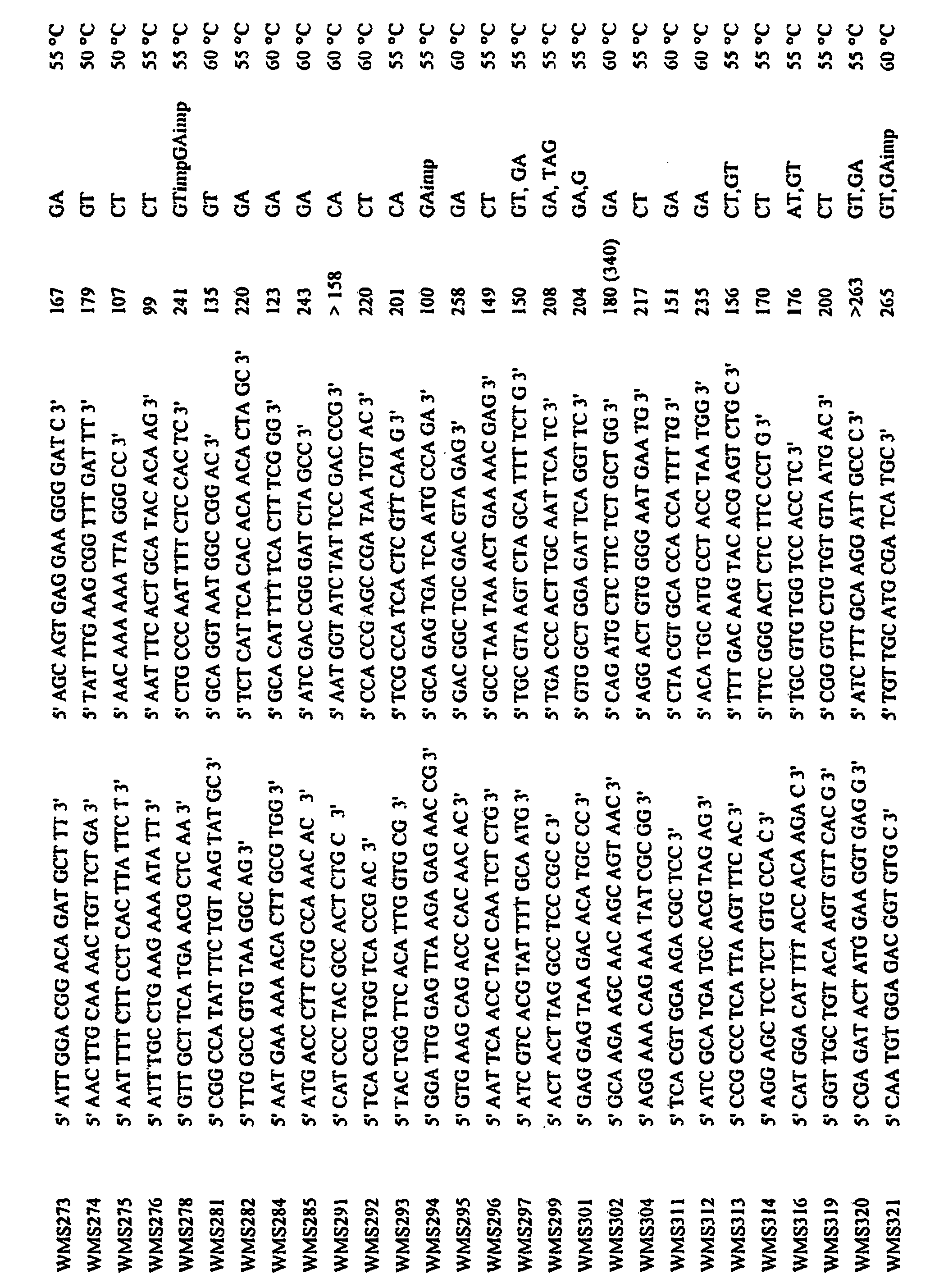

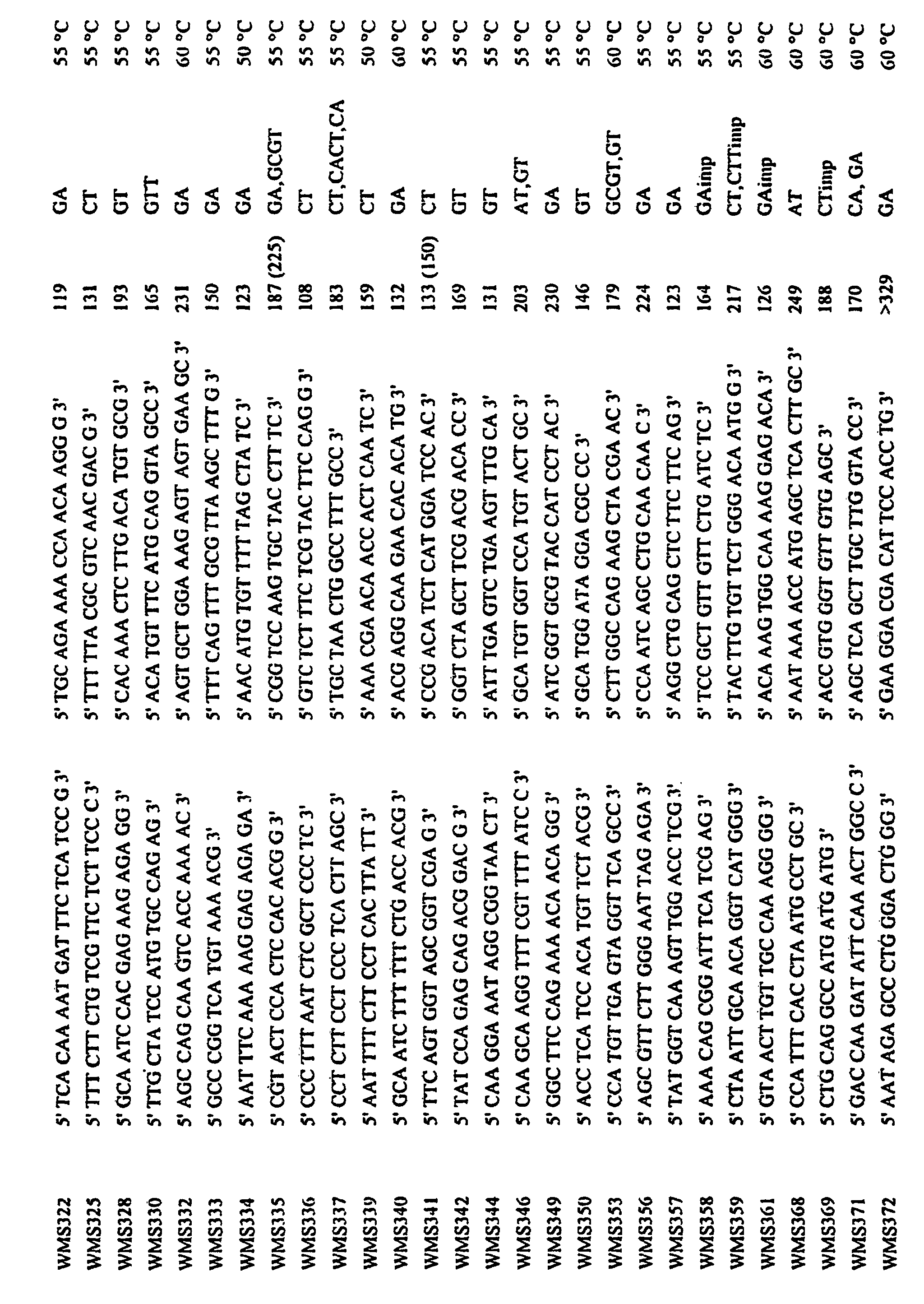

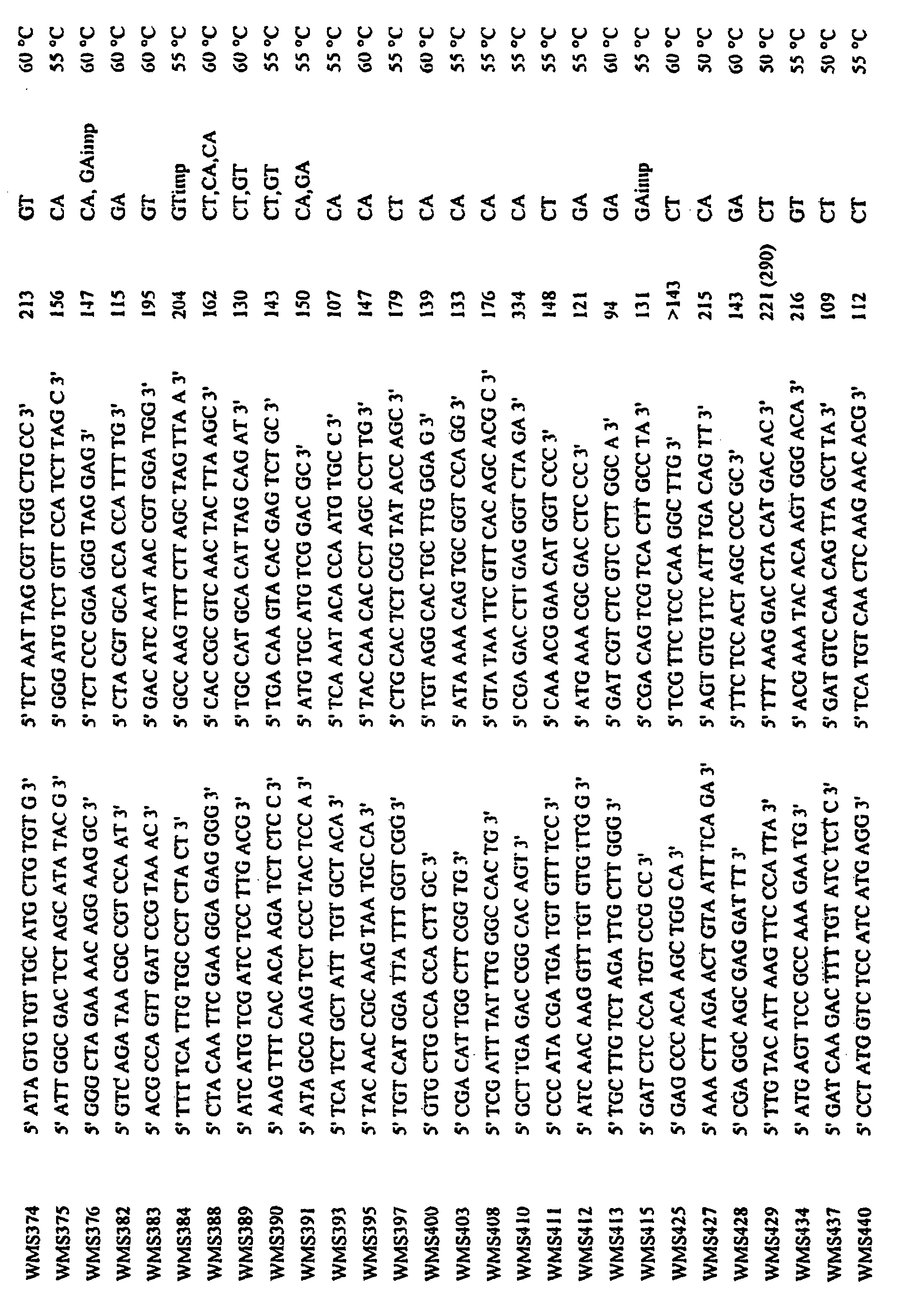

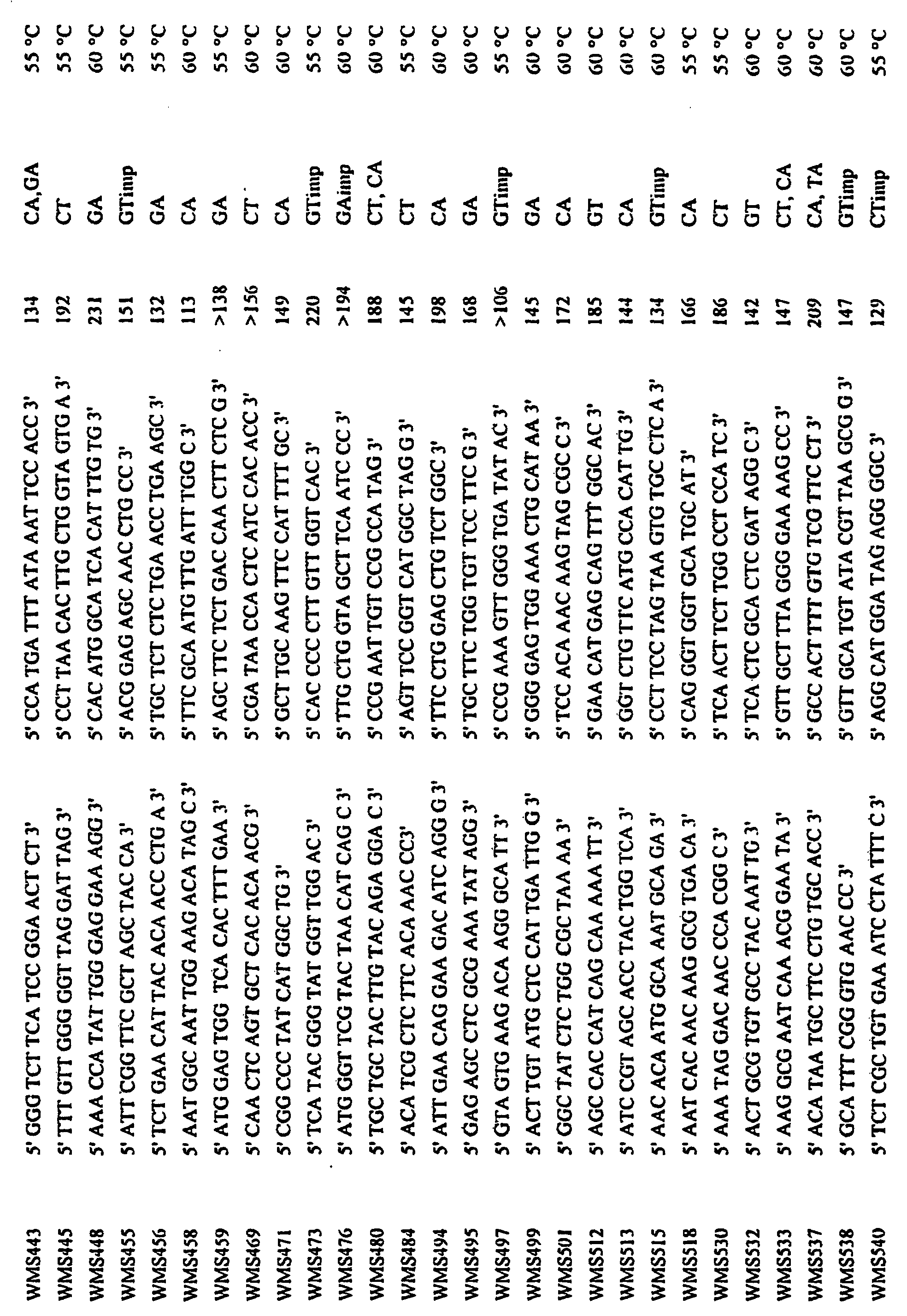

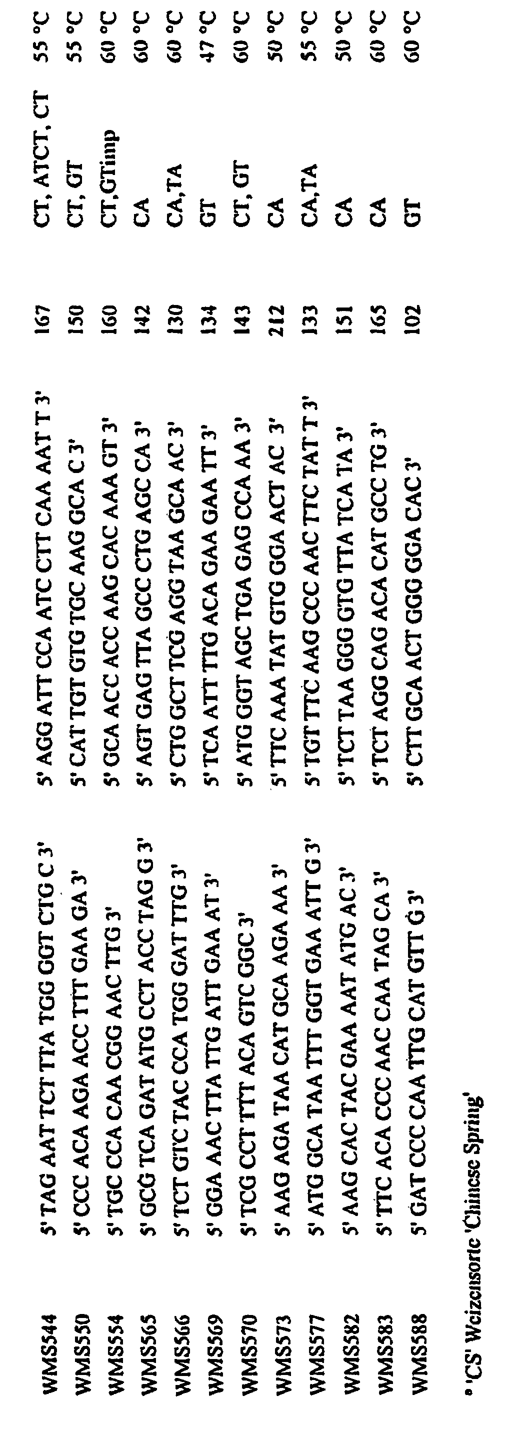

Diese Marker zeichnen sich durch einen hohen Grad an Polymorphismus zwischen verschiedenen Weizensorten bzw. - linien aus und detektieren in der Regel in verschiedenen Weizenlinien mehrere Allele pro genetischen Locus.

Sie sind daher für 'DNA fingerprinting', Sortenidentifikation, Verwandschafts- bzw. Ähnlichkeitsstudien, Charakterisierung von cytologischen Linien wie z. B. Deletionslinien, Substitutionslinien, Additionslinien etc. und allen Formen von genetischen Kartierungen, einschließlich der Kartierung von Einzelgenen und quantitativen Merkmalen (QTLs), verwendbar. Außerdem ist ihr Einsatz sehr gut für eine Automatisierung geeignet und es ist möglich, die Detektion der Produkte mit nichtradioaktiven Methoden durchzuführen.

Mit Hilfe dieser erfindungsgemäßen Marker ist z.B. die Möglichkeit einer Unterscheidung nahezu aller europäischen Weizenlinien gegeben.

Anschließend wird die Erfindung an Ausführungsbeispielen näher erläutert.

### 1. Amplifikation der Mikrosatellitenmarker

Die Amplifikation der Mikrosatellitenmarker erfolgt nach folgendem Protokoll:
10 mM Tris-HCl pH 8
50 mM KCl
1,5 mM MgCl₂ (in wenigen Ausnahmen 3 mM MgCl₂)
0,01% (w/v) Gelatine
0,2 mM von jedem Deoxynukleotid
250 nM von jedem Primer (jeweils der linke und der rechte
von einem Paar)
1-2 U *Taq*-Polymerase
50-150 ng Matrizen (template) DNS
werden in einem Volumen von 25 oder 50 µl nach folgendem Profil amplifiziert:

| | | |
|---|---|---|
| 92 °C | 3 min. | |
| | | |
| 92 °C | 1 min (Denaturierungsphase) | |
| 60 °C | 1 min. (Annealing-Phase) | 45 Zyklen |
| 72 °C | 2 min (Elongationsphase) | |
| | | |
| 72 °C | 10 min (Extensionsphase) | |

Die Amplifikation erfolgt in einem Perkin Elmer 9600 mit Deckelheizung oder in einem MJ Research Thermocycler ohne Deckelheizung. In diesem Gerät werden die Reaktionen mit Mineralöl überschichtet. Die Temperatur der Annealingphase richtet sich nach dem Schmelzpunkt (Tₘ) der Primer und beträgt in manchen Fällen auch 50 °C oder 55 °C.

### 2. Auftrennung der Mikrosatellitenmarker auf nicht denaturierenden Polyacrylamidgelen

Die PCR-Reaktionen werden mit 1/10 Vol. Stop-Puffer (0,02 M Tris-Acetat pH 8,1, 0,025 M Natrium-Acetat, 0,02 M EDTA, 70 % Glycerin, 0,2 % SDS, 0,6 % Bromphenolblau, 0,6 % Xylencyanol) versetzt und jeweils 25 µl werden in 10 %igen Polyacrylamidgelen (1,5 mm dick, 18 cm lang) aufgetrennt.

Ansatz für 1 Polyacrylamidgel (10%):

| | |
|---|---|
| 25 ml | Acrylamidstammlösung (19 g Acrylamid, 1 g Bisacrylamid, auf 100 ml mit H₂O) |
| 10 ml | 5X TBE (1X TBE = 0,09 M Tris-Borat pH 8,3, |
| | 0,002 M EDTA) |
| 15 ml | H₂O |

werden gemischt und die Polymerisierung durch Zugabe von 220 µl Ammoniumpersulfat (10 %, frisch angesetzt) und 20 µl TEMED gestartet. Sofort nach Zugabe wird das Gemisch in die abgedichtete Gelform gegossen und der Kamm zur Taschenbildung eingesetzt. Nach ca. 1 h ist die Polymerisierung abgeschlossen. Das Gel wird in die Gelkammer eingesetzt und es erfolgt ein Vorlauf ohne Proben für circa 30 min. bei 150 Volt in 1X TBE. Danach werden die Proben geladen (je 25 µl) und bei 100 Volt 14-16 h lang aufgetrennt.

Nach Beendigung der Elektrophorese wird das Gel für circa 20 min. in Ethidiumbromid (1-2 Tropfen 10 mg/ml in 11 H₂O) gefärbt und die Fragmente durch einen UV-Transilluminator sichtbar gemacht und dokumentiert.

### 3. Auftrennung der Mikrosatellitenmarker auf denaturierenden Gelen

Zur Auftrennung der amplifizierten Fragmente auf denaturierenden Gelen wird beispielsweise ein Automatischer Laser Fluorescence (A.L.F.) Sequencer (Pharmacia) genutzt. Um die Detektion der Fragmente mittels Laser zu ermöglichen wird ein Primer je Paar am 5' Ende fluorescein-markiert. Je 0,3 bis 1,5 Mikroliter pro PCR-Reaktion werden mit 2,5 Mikrolitern Stop-Puffer (deionisiertes Formamid; 5 mg/ml Dextran Blue) versetzt, denaturiert (1 min; 90°C) und auf das Gel geladen. Es werden Gelplatten mit 9 cm Auftrennungsdistanz genutzt, wie vom Hersteller für die ragmentanalyse empfohlen. Die Gellösung beinhaltet 6,5 % Long-Ranger (AT Biochem), 7M Harnstoff und 1,2X TBE-Puffer. Die Gele sind 0,35 oder 0,5 mm stark. Die Konditionen für Gellauf sind 600 V, 40 mA, 50 W, 0,84 s Datensammlungs-Intervall und 2 mW Laser-Energie. Die Gelläufe werden nach ca. 80-90 min beendet, was für eine Detektion von Fragmenten bis zu 300 bp Größe ausreicht. Ein Gel kann für vier bis fünf Läufe verwendet werden. Für jeden Gellauf wird eine Datendatei erhalten. Mit dieser Datei und anhand interner Größenstandards werden im Computer-Programm Fragment-Manager (Pharmacia) die genauen Fragmentgrößen ermittelt, und somit kleinste Größenunterschiede von einem Basenpaar bestimmt.

## Patentansprüche

1. Ein Satz von Mikrosatellitenmarker (auf der Basis hypervariabler Genomabschnitte) für Pflanzen der Spezies Triticum aestivum, sowie des Tribus Triticeae unter Verwendung der Polymerasekettenreaktion (PCR), gekennzeichet durch eine sequence tagged site (STS), die durch zwei spezifische Primer definiert ist, die jeweils spezifisch zu einer Mikrosatellitensequenz sind und im Durchschnitt 20 ± 3 Basen lang sind und links und rechts eine Mikrosatellitensequenz flankieren, wobei jeder Marker durch Amplifikation einer Mikrosatellitensequenz mittels PCR zu Fragmenten verschiedener Länge führt und wobei die Primerpaare aus der nachstehenden Liste ausgewählt werden

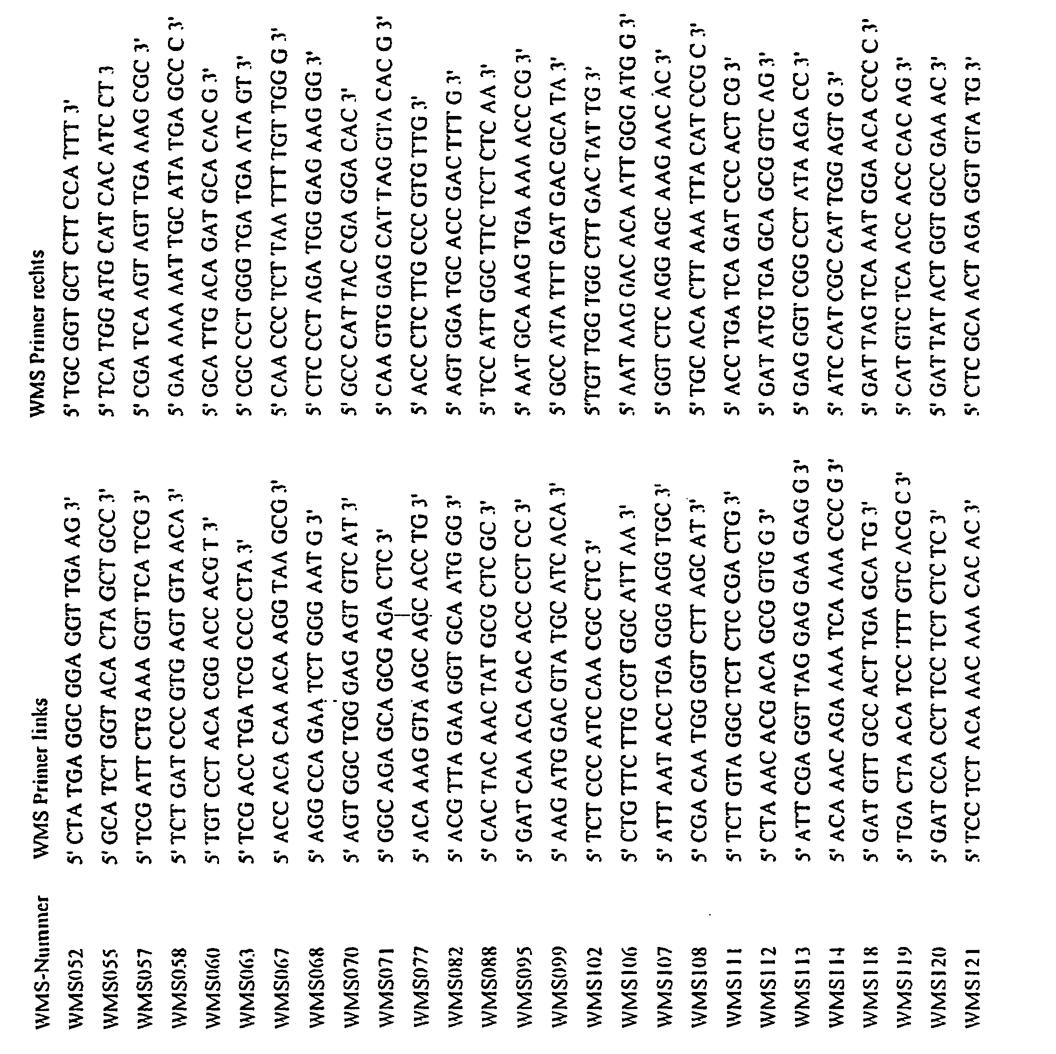

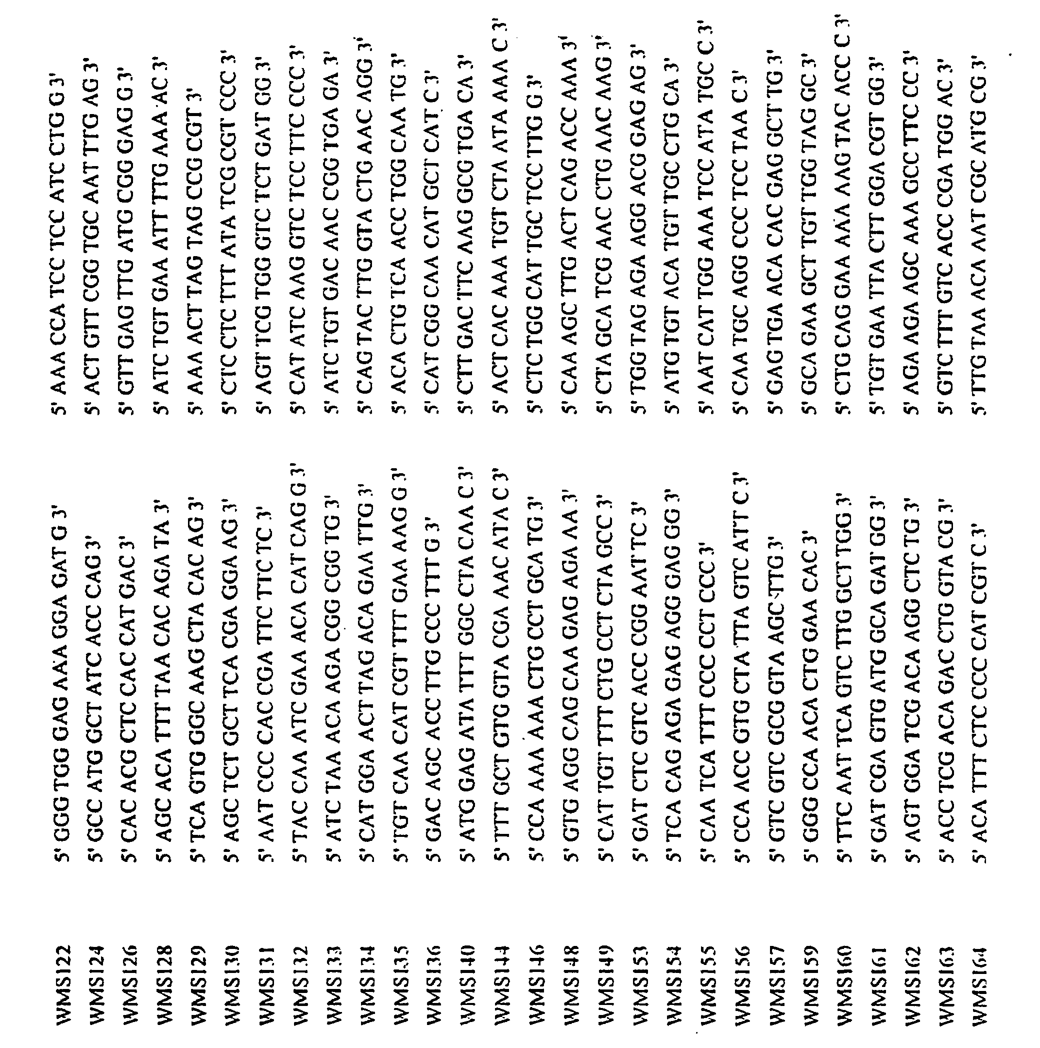

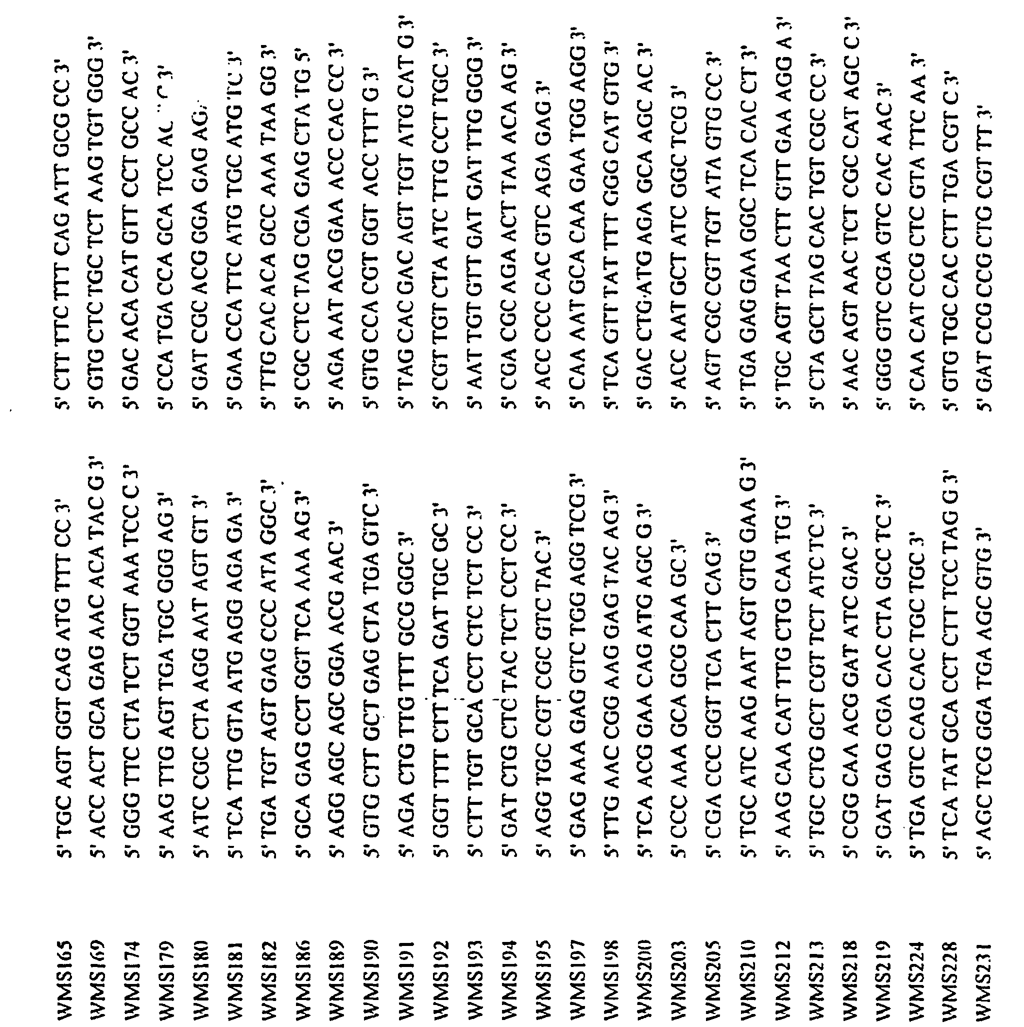

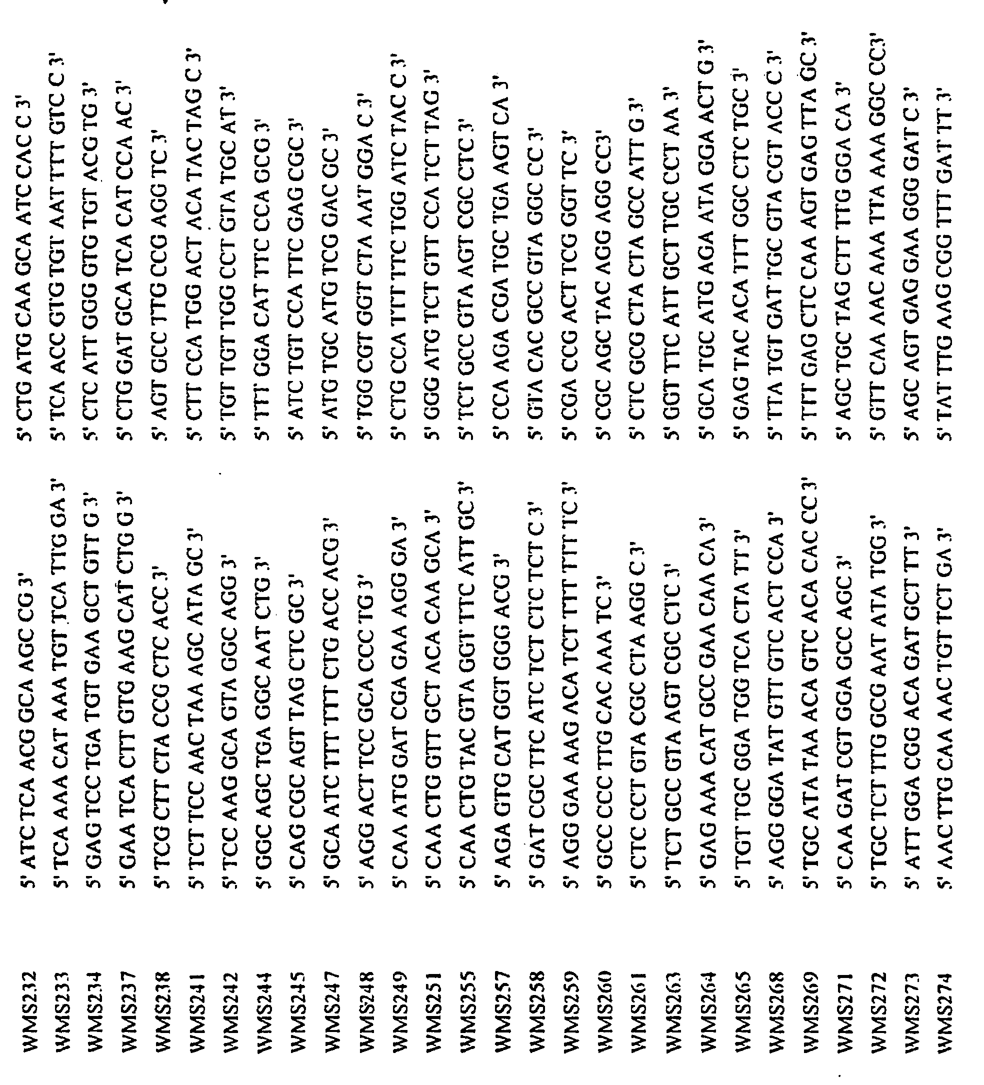

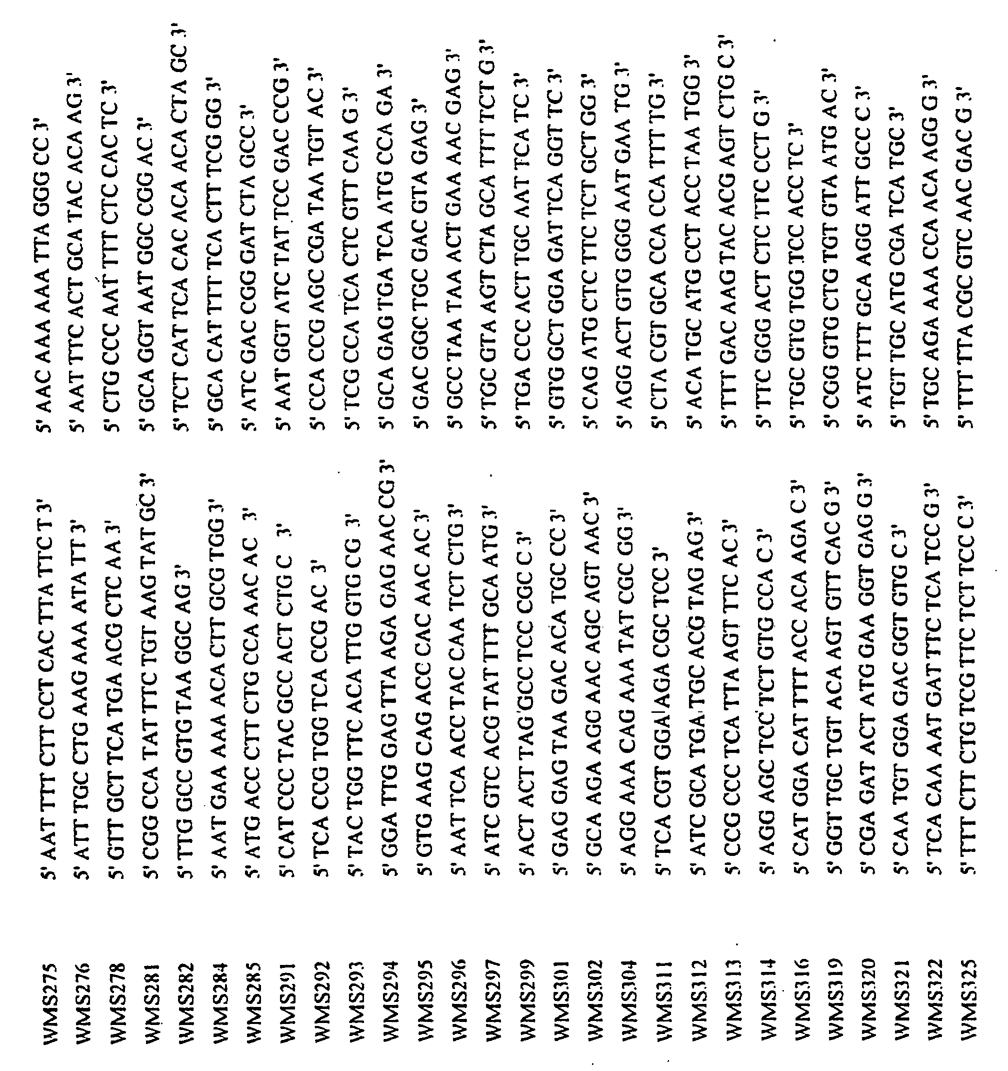

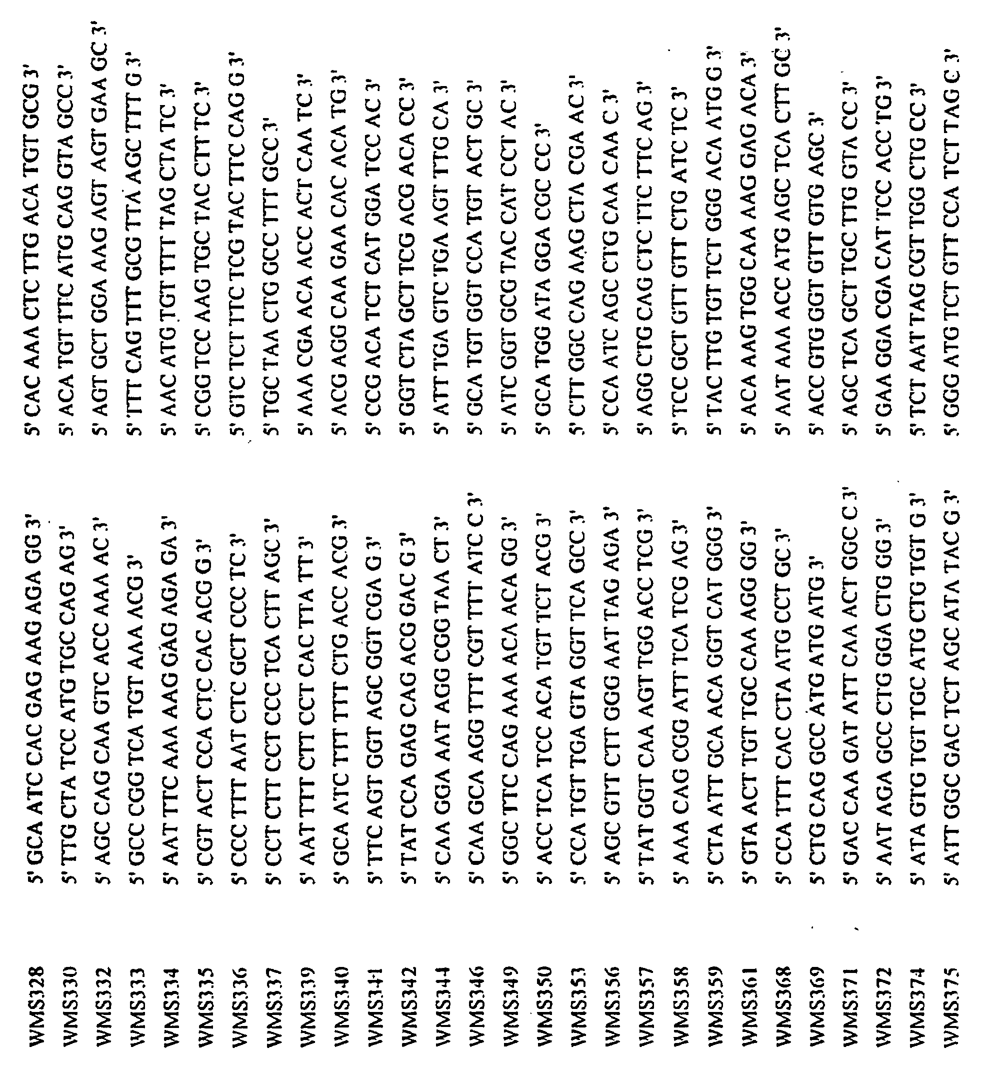

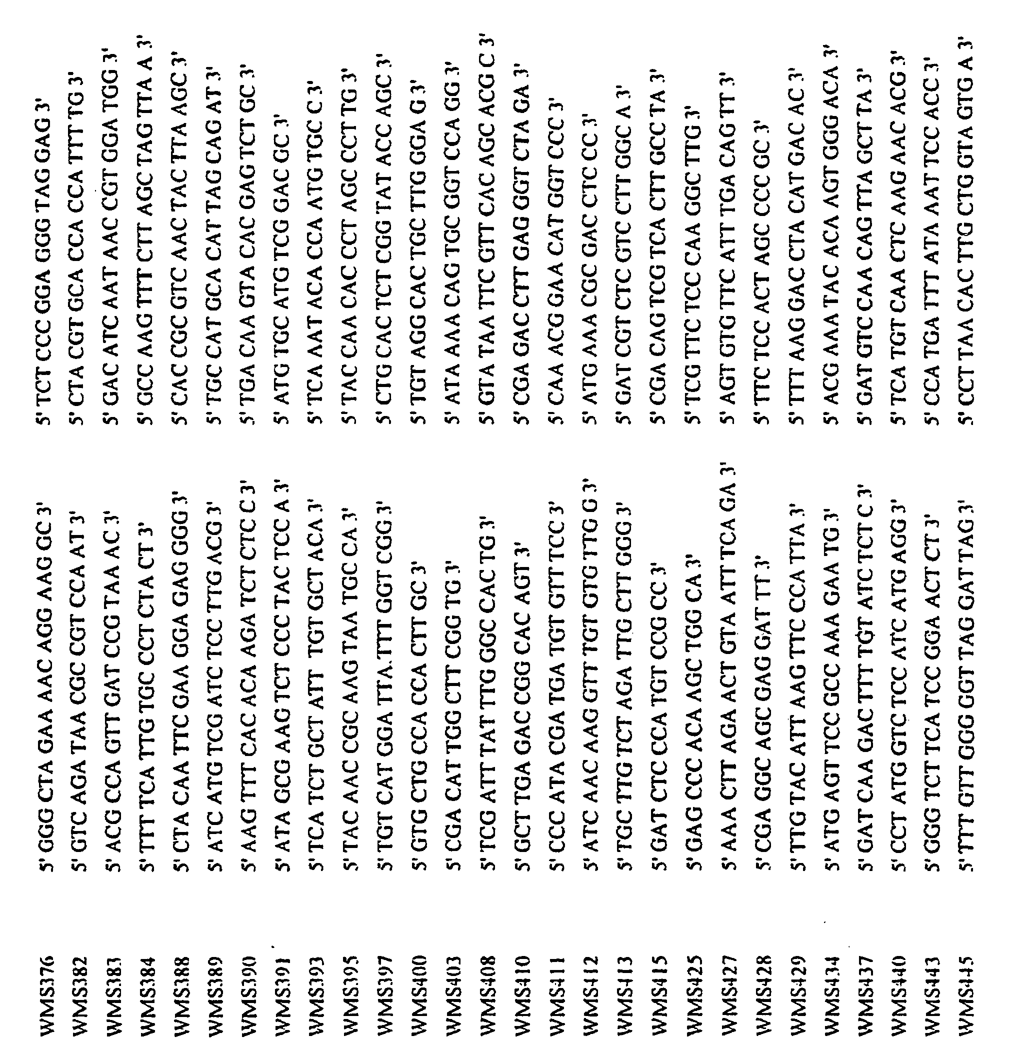

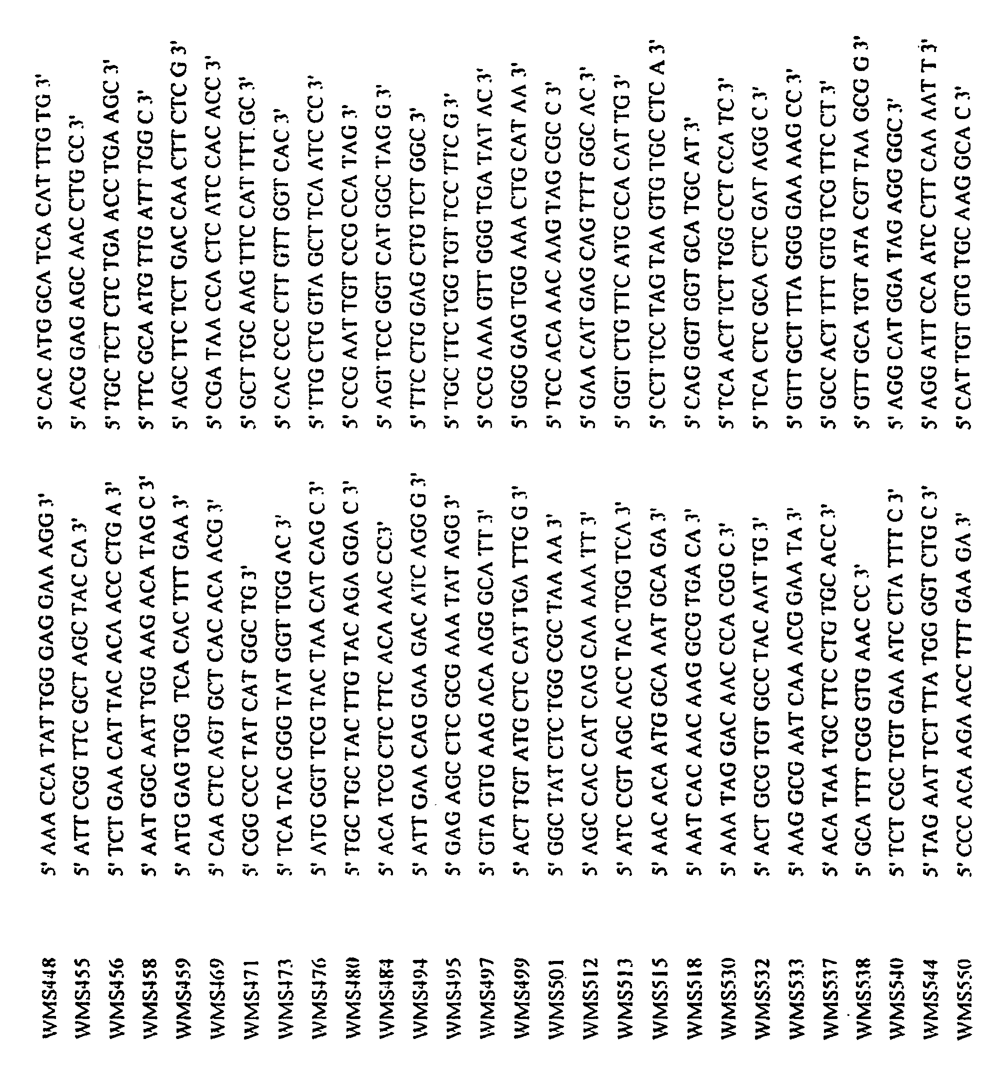

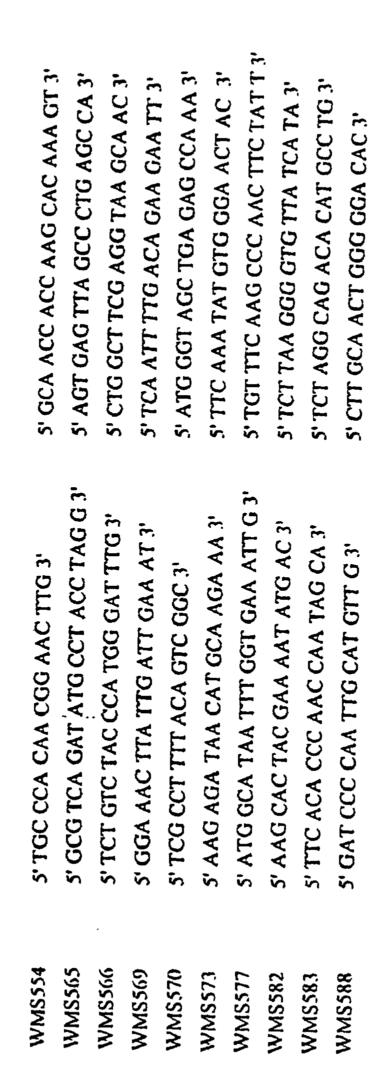

2. Verfahren zur Herstellung eines Mikrosatellitenmarkers gemäß Anspruch 1 für Pflanzen der Spezies Triticum aestivum, sowie des Tribus Triticeae, **dadurch gekennzeichnet, daß** hypervariable Genomabschnitte (sogenannte Mikrosatelliten) mit Hilfe der Polymerasekettenreaktion (PCR) zu polymorphen Fragmenten in Gegenwart zweier spezifischer Primer aus der Liste der Primerpaare nach Anspruch 1, die links und rechts für jeden Mikrosatelliten-Locus eine Mikrosatellitensequenz flankieren, amplifiziert, anschließend aufgetrennt und detektiert werden.

3. Verfahren nach Anspruch 2, **dadurch gekennzeichnet, daß** zur Auftrennung der Marker hochauflösende Agarosegele, native Polyacrylamidgele oder denaturierende Polyacrylamidgele verwendet werden.

4. Verfahren nach Anspruch 2, **dadurch gekennzeichnet, daß** die Detektion je nach Trennungssystem über Ethidiumbromidfärbung, Silberfärbung, bei radioaktiver Markierung über Autoradiographie oder mittels automatischem Sequenziergerät unter Verwendung farbstoff- bzw. fluoreszenzmarkierter Primer erfolgt.

5. Verwendung der Mikrosatellitenmarker nach Anspruch 1 zur genetischen Analyse hexaploider und tetraploider Kulturformen des Weizens.

6. Verwendung nach Anspruch 4 zur genetischen Kartierung und Markierung von monogenen und polygenen Eigenschaften und deren Selektion, zur Verwandtschaftsanalyse und Sortenidentifikation sowie zur Evaluierung von Sortenreinheit, Hybrididentifikation und Pflanzenzüchtung.

## Claims

1. A set of microsatellite markers (on the basis of hypervariable genome sections) for plants of the Triticum aestivum species as well as of Tribus Triticeae making use of the polymerase chain reaction (PCR), wherein there exists a sequence tagged site (STS) defined by two specific primers, each of which is specific to a microsatellite sequence and has an average length of 20 ± 3 bases and flanks a microsatellite sequence on the left and right, with each marker leading to fragments of various lengths by amplification of a microsatellite sequence by means of PCR and with the primary pairs being selected from the following list:

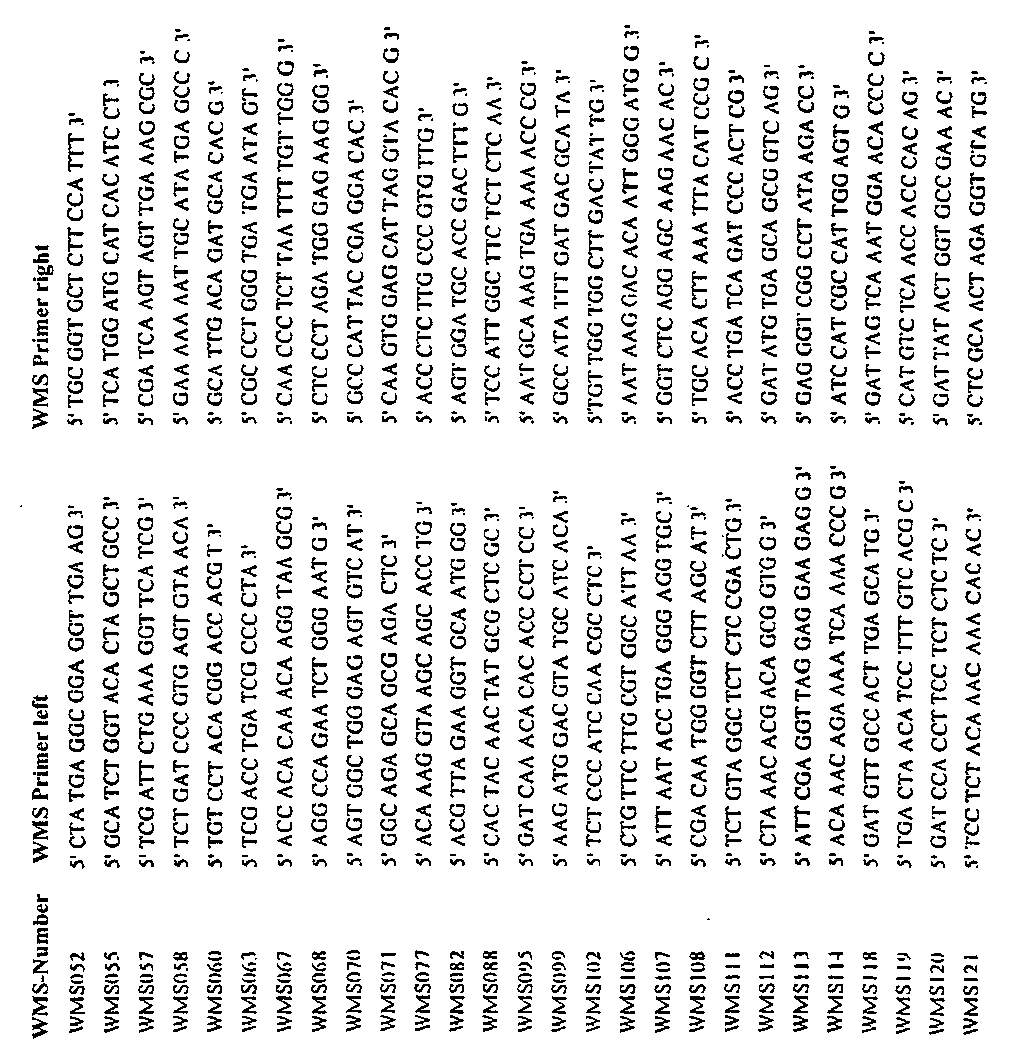

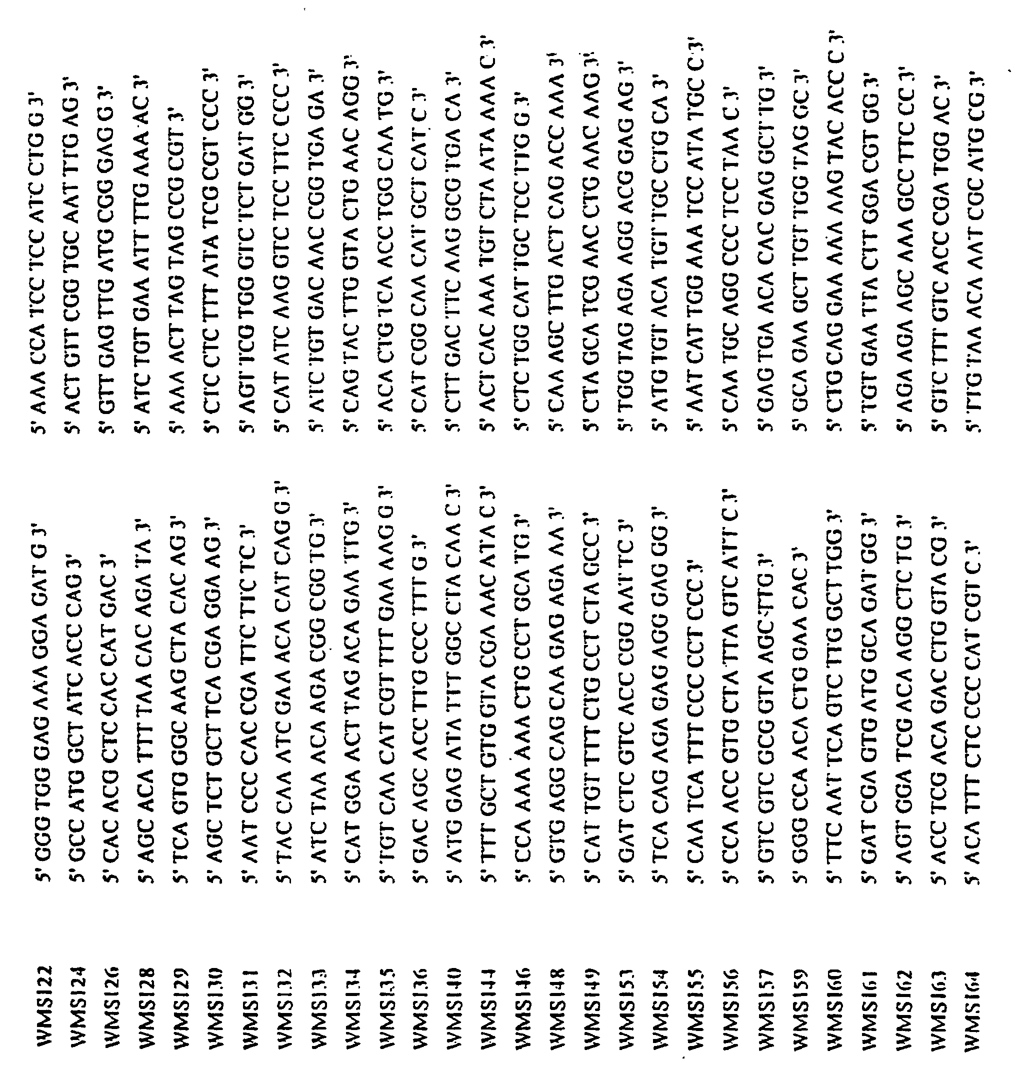

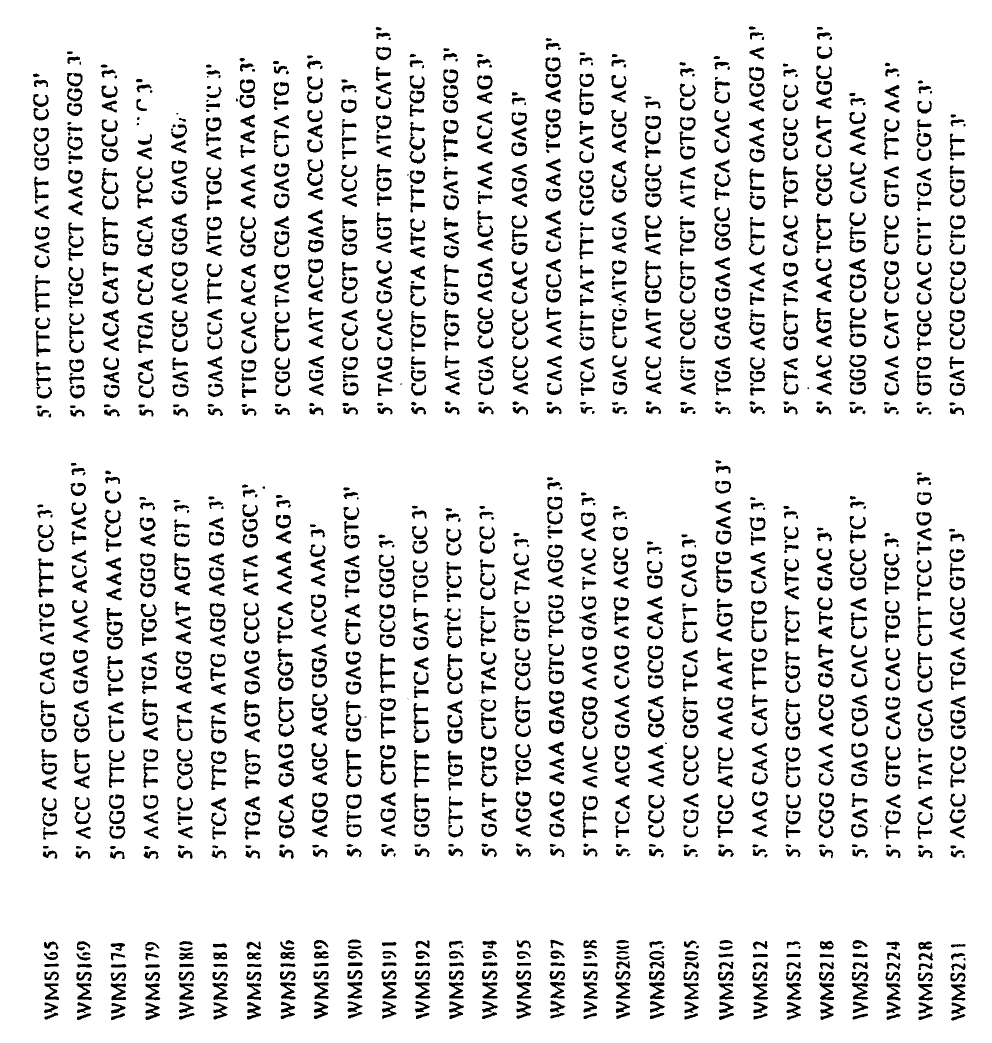

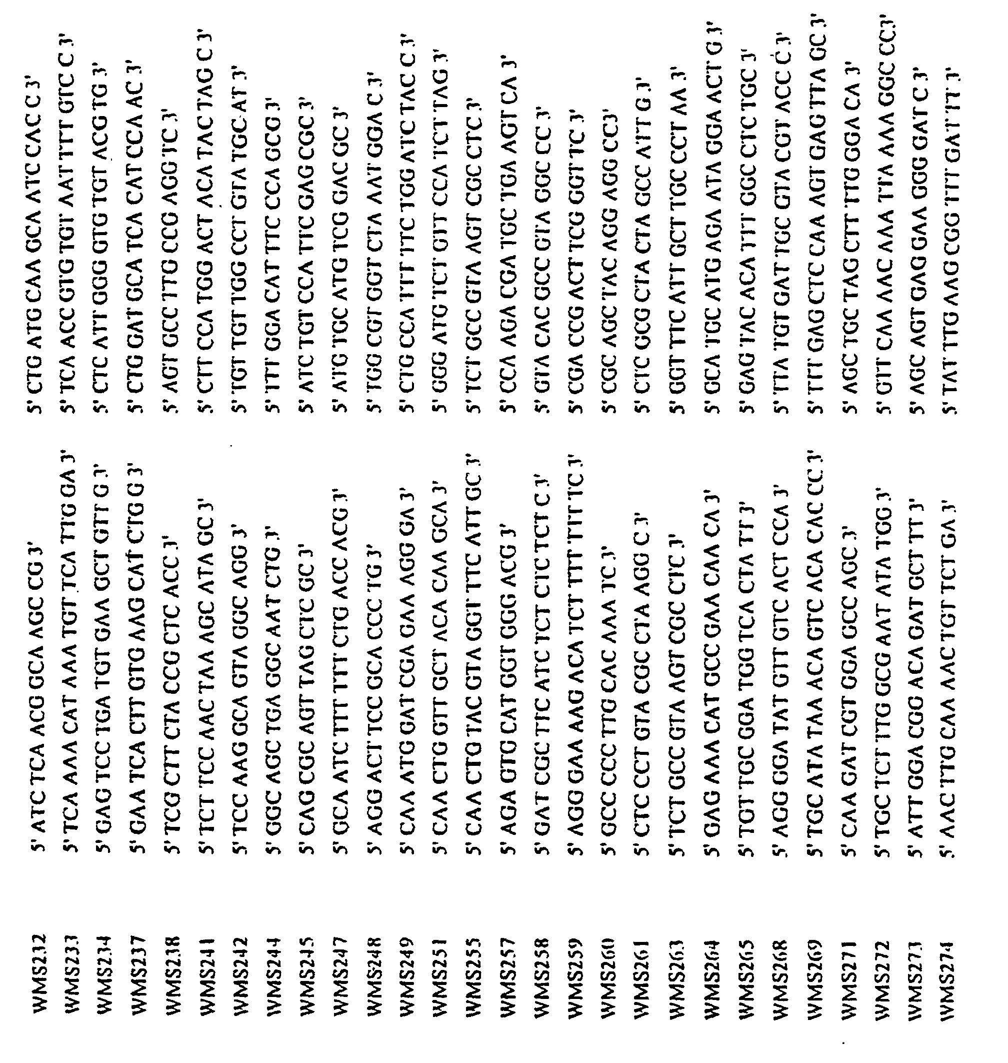

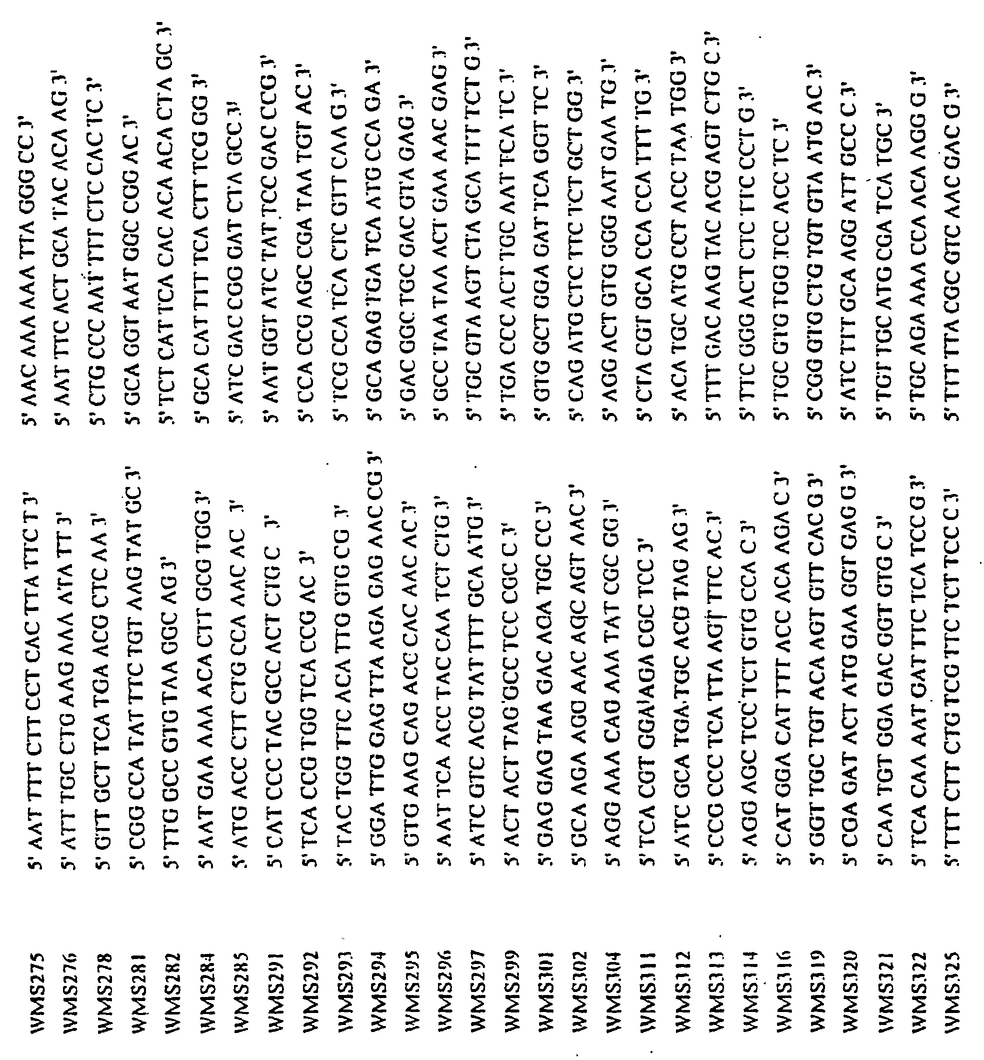

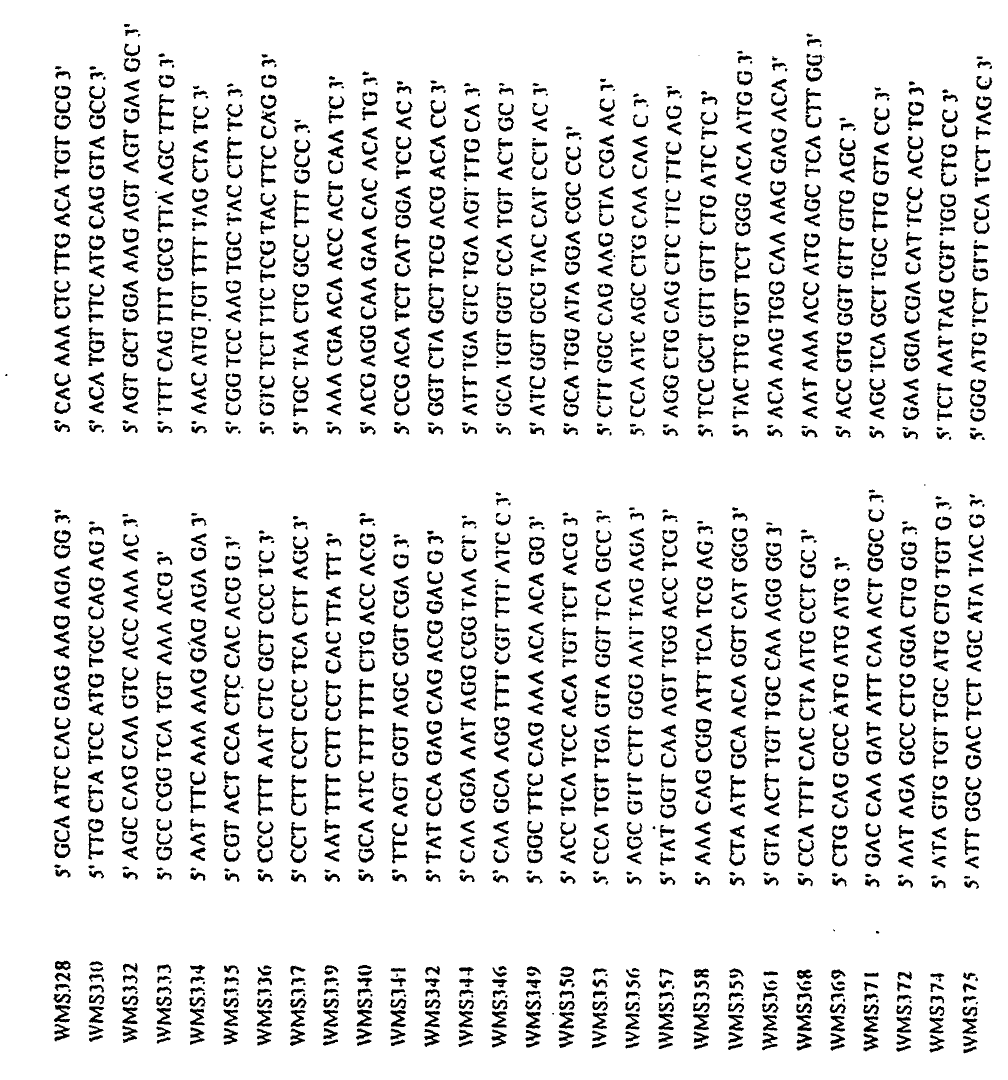

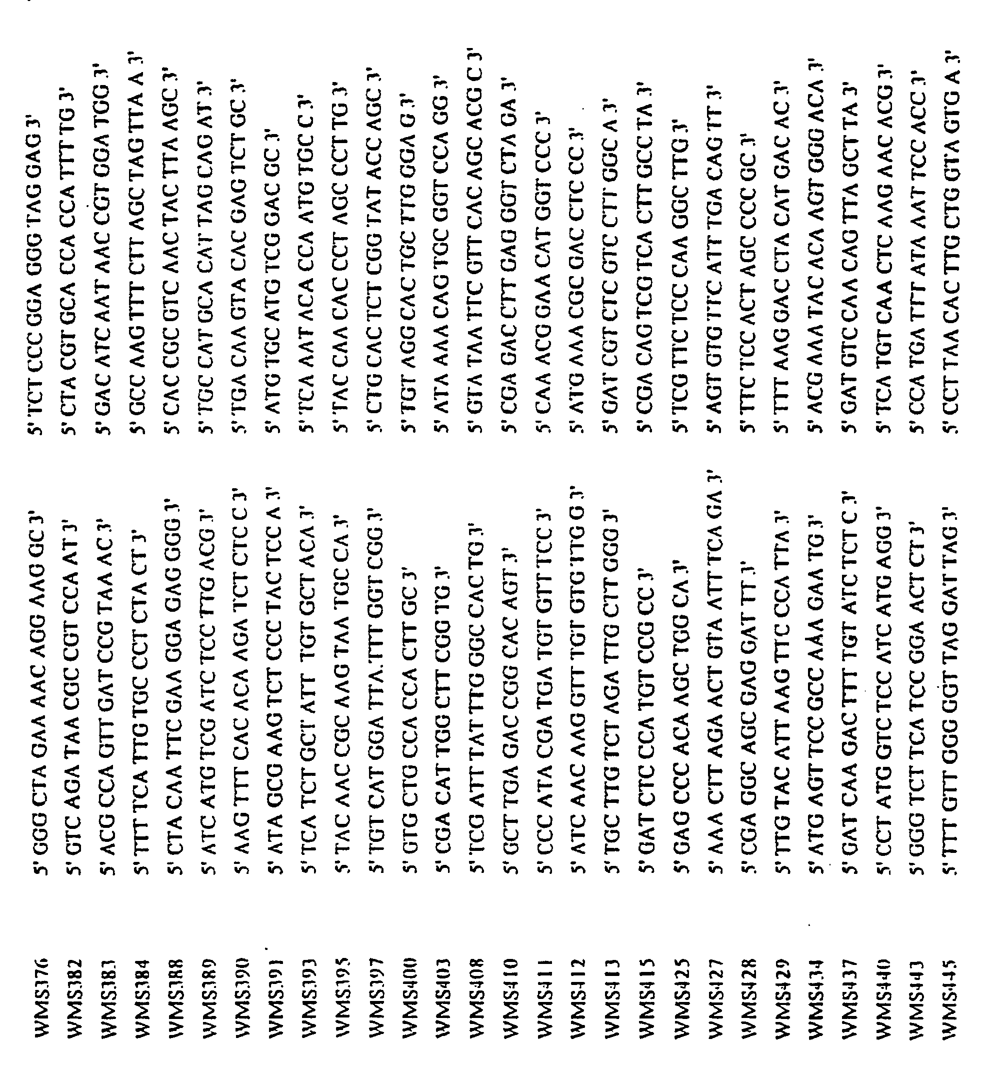

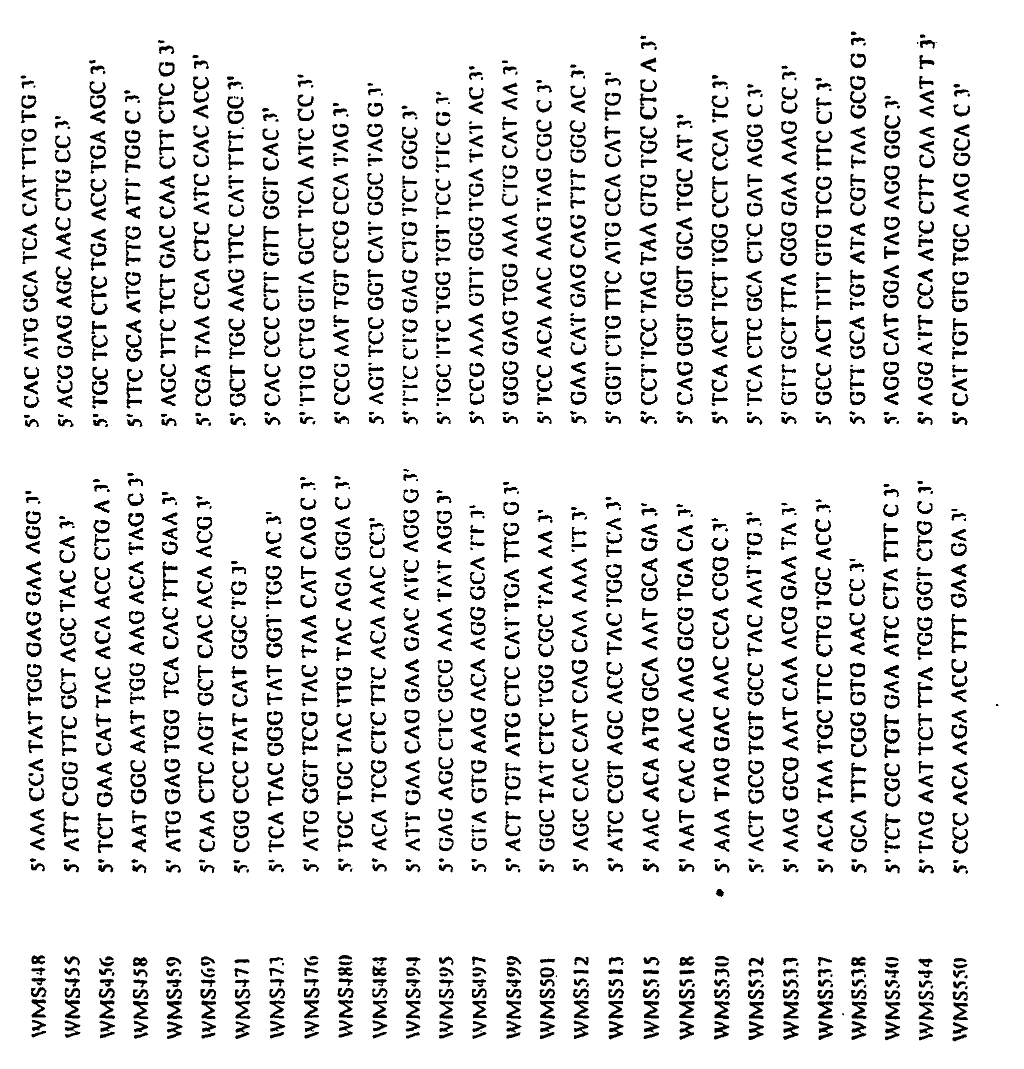

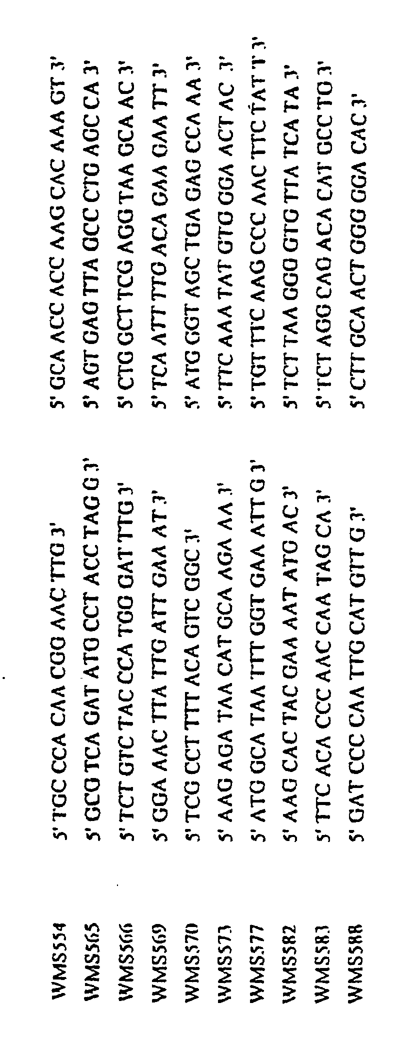

2. Method for the production of a microsatellite marker according to Claim 1 for plants of the Triticum aestivum species as well as of Tribus Triticeae, wherein hypervariable genome sections (so-called microsatellites) are amplified with the help of the polymerase chain reaction (PCR) to form polymorphous fragments in the presence of two specific primers from the list of primary pairs according to Claim 1, which flank a microsatellite sequence on the left and right for each microsatellite locus, these sections subsequently being separated and detected.

3. Method according to Claim 2, wherein high-resolution agarosegels, native polyacrylamide gels or denaturising polyacrylamide gels are used for the separation of the markers.

4. Method according to Claim 2, wherein the detection is done via ethidium bromide staining, silver staining, via auto-radiography for radioactive marking or by means of an automatic sequencing appliance making use of dry or fluorescence-marked primers, depending upon the separation system.

5. Use of the microsatellite markers according to Claim 1 for the genetic analysis of hexaploid and tetraploid culture forms of wheat.

6. Use according to Claim 4 for the genetic mapping and marking of monogenic and polygenic properties and their selection, for relationship analysis and sort identification and for the evaluation of sort purity, hybrid identification and plant breeding.

## Revendications

1. Un lot de marqueurs microsatellites (sur la base de sections de génomes hypervariables) pour les plantes de l'espèce Triticum aestivum ainsi que du Tribus triticeae sous application de la réaction en chaîne de polymérase (PCR), se caractérisant par une sequence tagged site (STS) qui est définie par deux primeurs qui sont spécifiques à chaque séquence microsatellite et sont en moyenne de 20 ± 3 bases de long et flanquent à gauche et à droite une séquence de microsatellite, chaque marqueur menant à des fragments de longueurs différentes par amplification d'une séquence de microsatellite au moyen de la réaction PCR et les couples de primeurs étant sélectionnés dans la liste ci-contre.

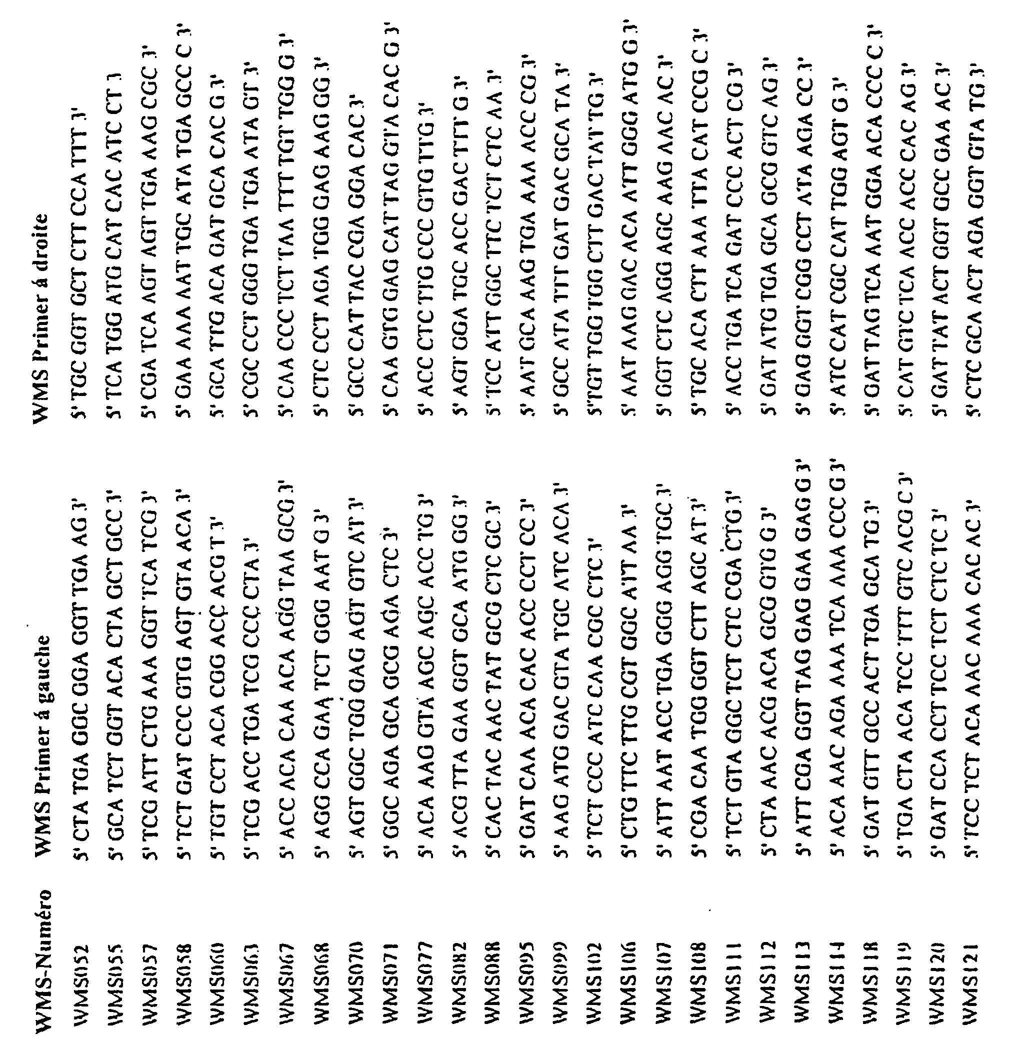

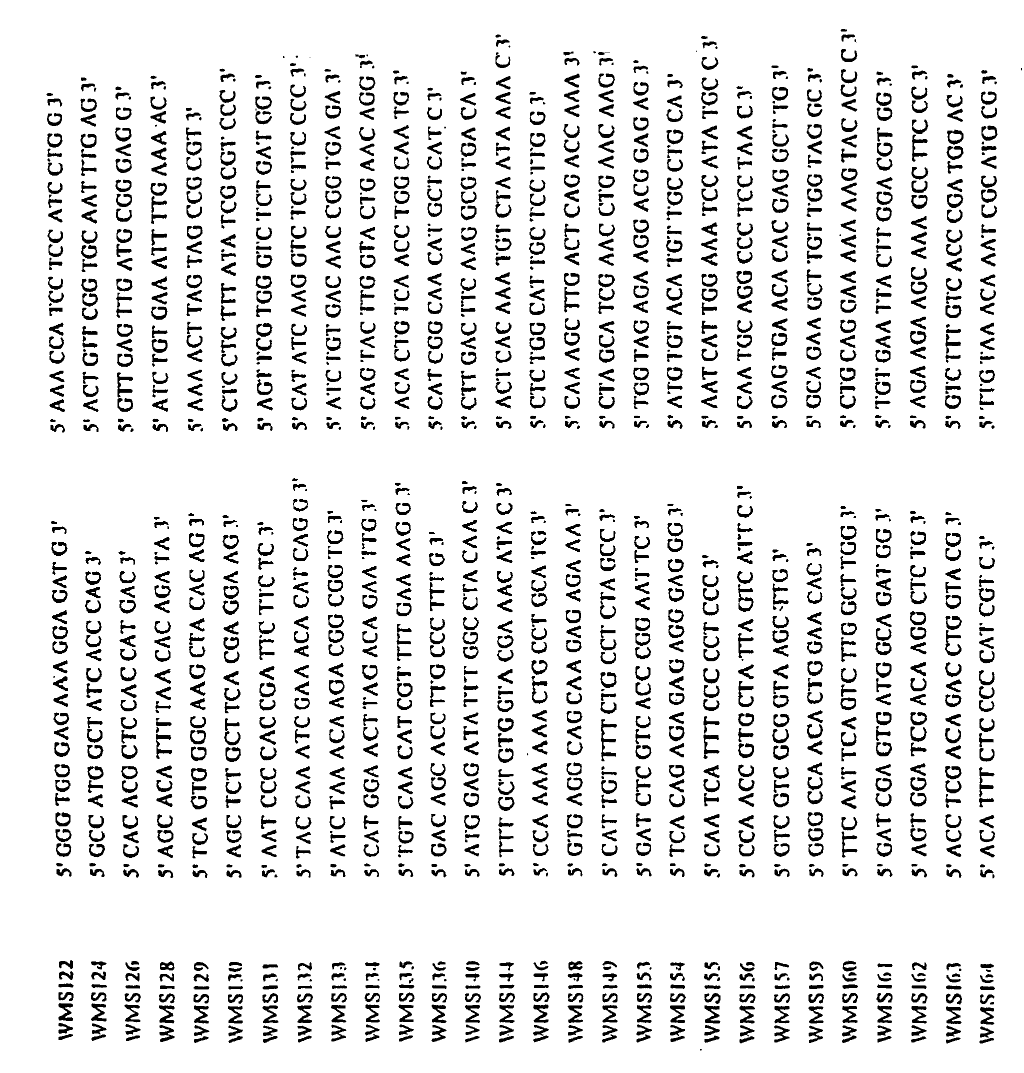

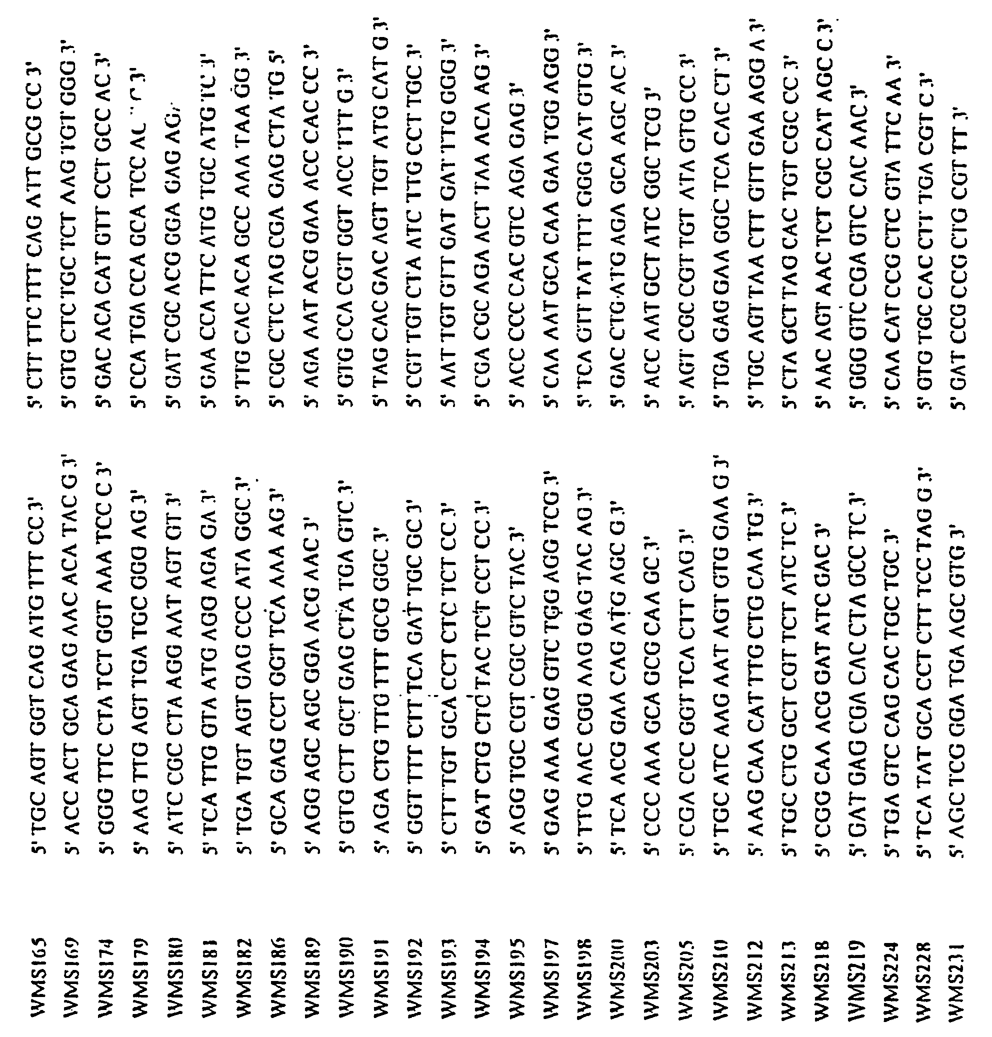

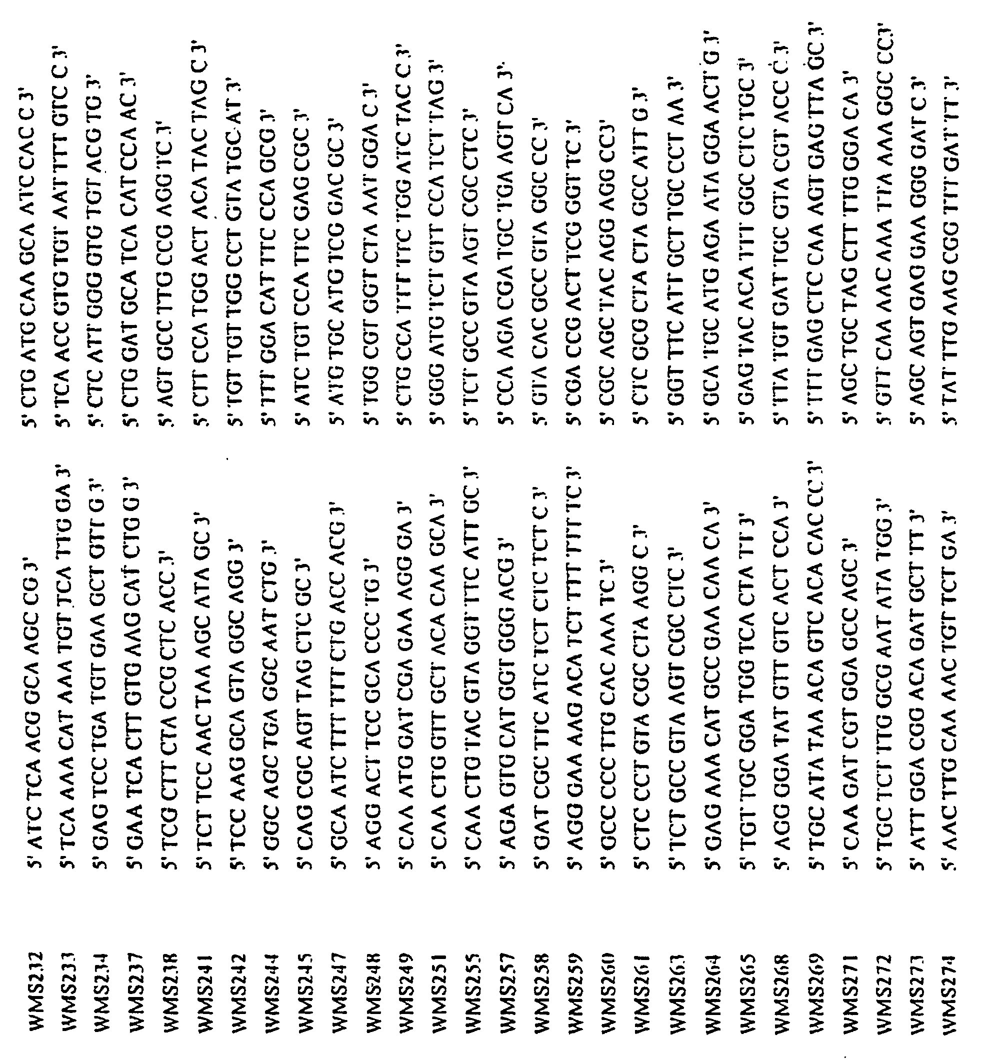

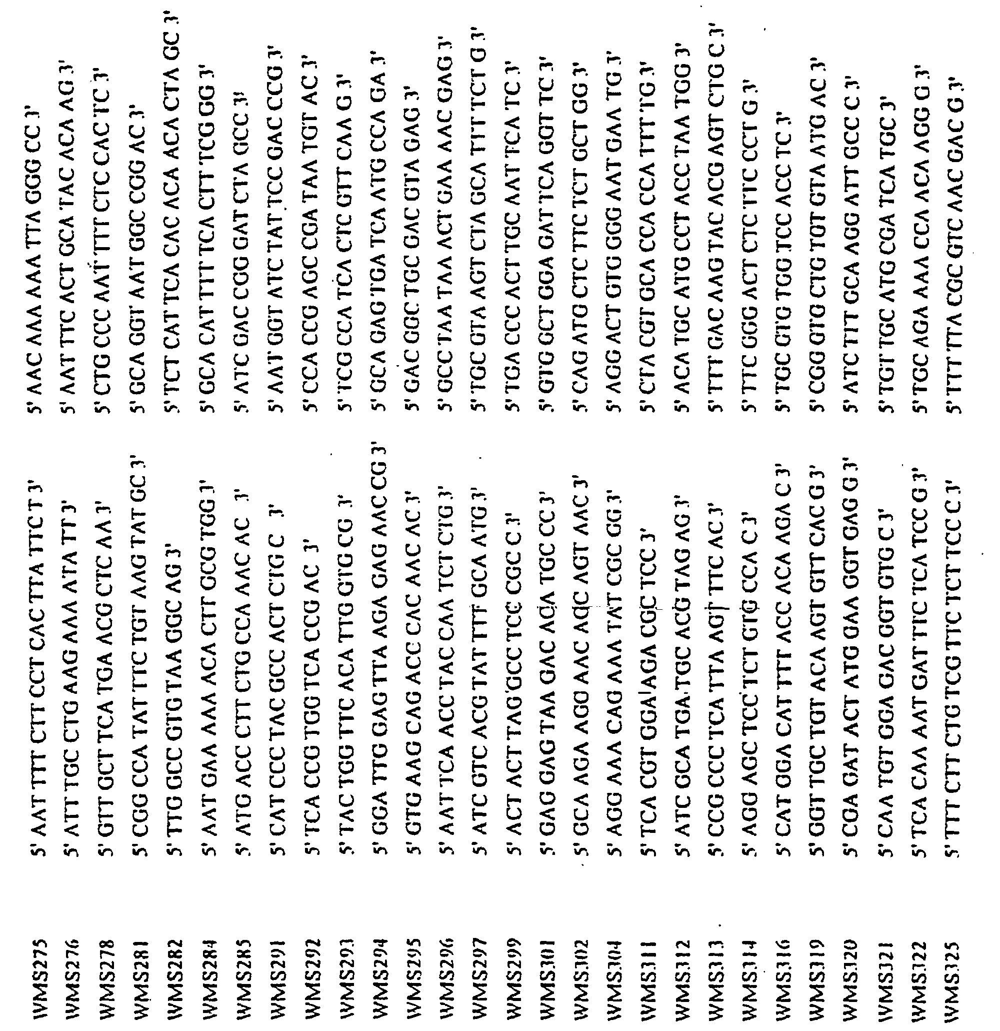

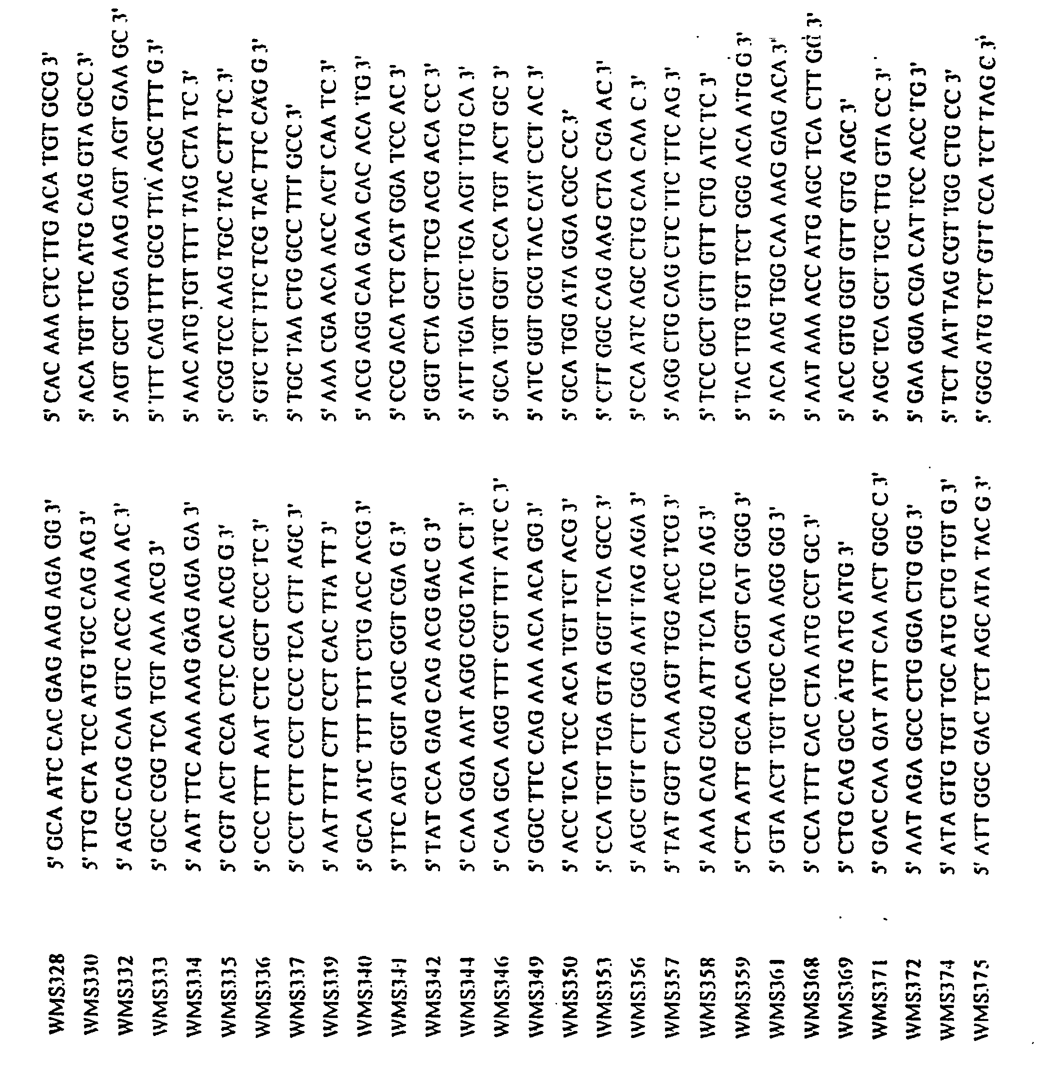

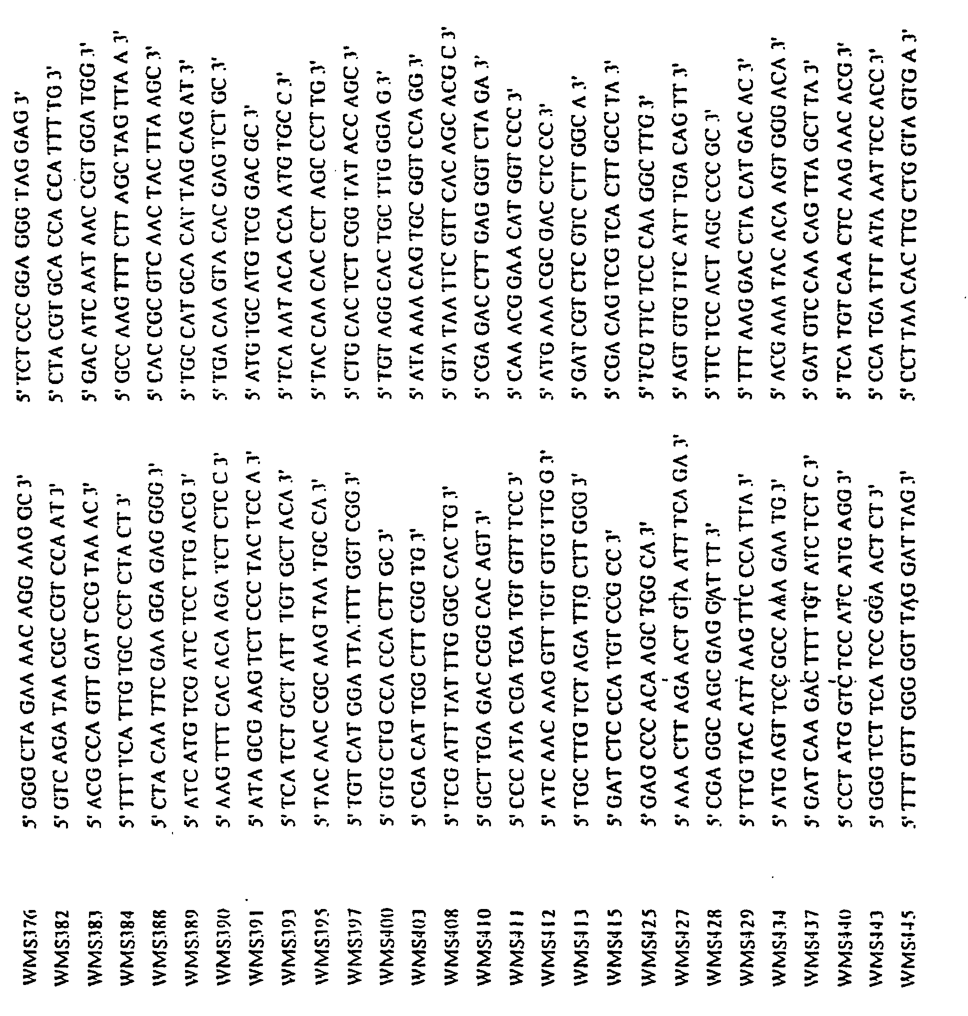

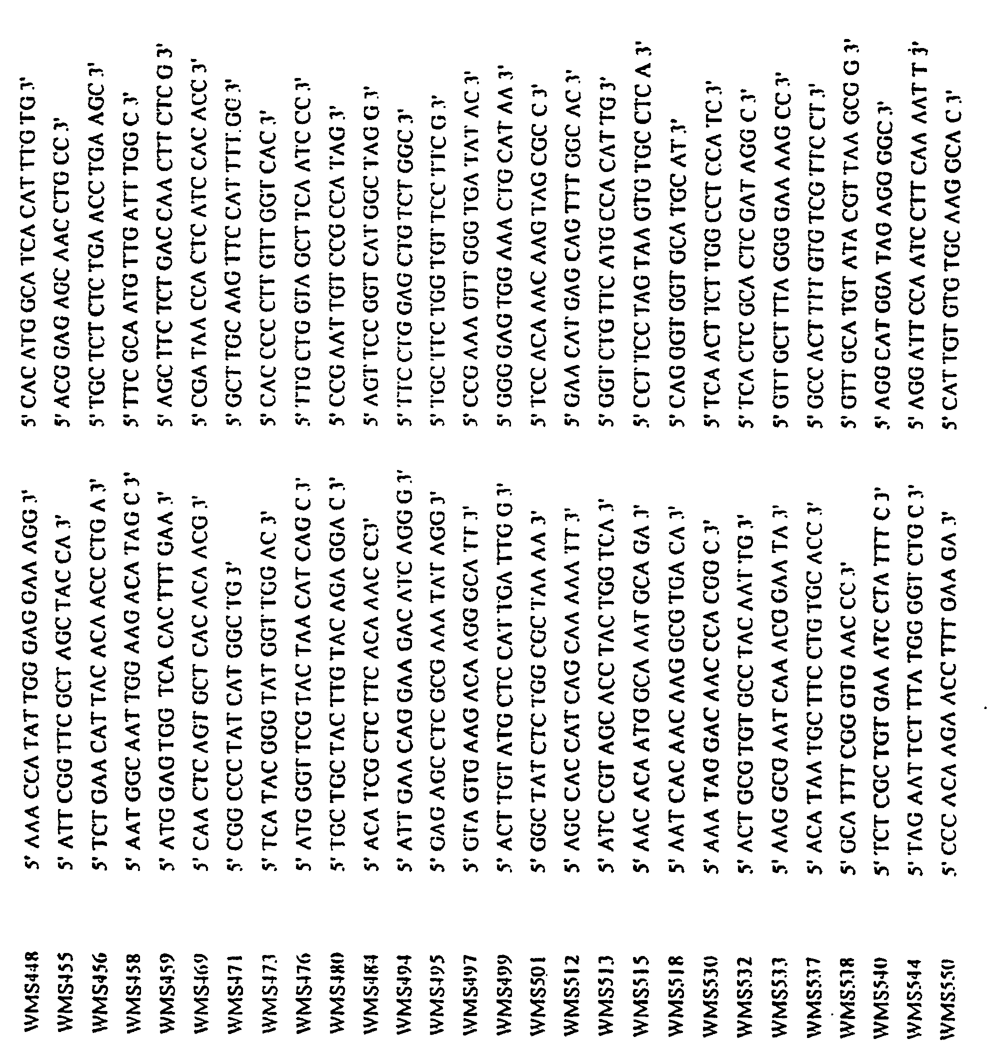

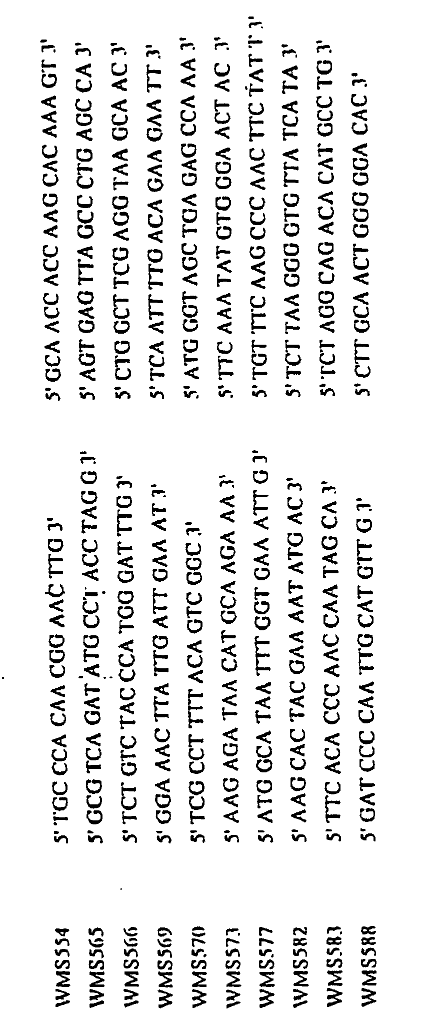

2. Procédé de fabrication d'un marqueur microsatellite conformément à la revendication 1 pour les plantes de l'espèce Triticum aestivum ainsi que du Tribus triticeae se caractérisant par le fait que des sections de génome hypervariables (dites microsatellites) sont amplifiées à l'aide de la réaction en chaîne de polymérase (PCR pour former des fragments polymorphes en présence de deux primeurs spécifiques, sélectionnés dans la liste des couples de primeurs selon la revendication 1 et qui flanquent à gauche et à droite une séquence de microsatellite pour chaque locus de microsatellite, sont ensuite séparées et détectées.

3. Procédé selon la revendication 2, se caractérisant par le fait que des gels d'agarose de haute résolution, des gels d'amide polyacrylique natifs ou des gels d'amide polyacrylique dénaturants sont employés pour séparer les marqueurs.

4. Procédé selon la revendication 2, se caractérisant par le fait que la détection est réalisée selon le système de séparation par coloration au bromide d'ethidium, coloration à l'argent, en cas de marquage radioactif par autoradiographie ou au moyen d'un appareil d'analyse séquentielle automatique sous application de primeurs marqués au colorant voire fluorescents.

5. Emploi des marqueurs microsatellites selon la revendication 1 pour l'analyse génétique de formes de cultures hexaploïdes et tetraploïdes du blé.

6. Emploi selon la revendication 4 pour la cartographie génétique et le marquage de propriétés monogènes et polygènes et leur sélection permettant de réaliser l'analyse de parenté et l'identification des sortes ainsi que l'évaluation de la pureté des sortes, l'identification des hybrides et la culture des plantes.
